Europäisches Patentamt

European Patent Office

Office européen des brevets

(19)

(11) Publication number: **0 079 373**
**B1**

## EUROPEAN PATENT SPECIFICATION

(12)

(45) Date of publication of patent specification: **30.12.86**

(51) Int. Cl.⁴: **A 61 F 2/22, A 61 M 1/12**

(21) Application number: **82901824.1**

(22) Date of filing: **12.05.82**

(86) International application number:
**PCT/US82/00629**

(87) International publication number:
**WO 82/03980 25.11.82 Gazette 82/28**

(54) **HYDRAULICALLY ACTUATED CARDIAC PROSTHESIS.**

(30) Priority: **19.05.81 US 265100**
**19.05.81 US 265199**

(43) Date of publication of application:
**25.05.83 Bulletin 83/21**

(45) Publication of the grant of the patent:
**30.12.86 Bulletin 86/52**

(84) Designated Contracting States:
**DE FR GB**

(56) References cited:
**FR-A-2 012 788**
**US-A-3 550 162**
**US-A-3 568 214**
**US-A-3 783 453**
**US-A-3 919 722**
**US-A-4 143 661**
**US-A-4 173 796**

**Transactions A.S.A.I.O., Volume XI, Issued
June 19, 1965, pages 265-268, "The
Development of an Electrohydraulic
Implantable Artificial Heart" by W.H. Burns et
al.**

(73) Proprietor: **THORATEC LABORATORIES
CORPORATION**
**2448 Sixth Street**
**Berkeley, CA 94710 (US)**

(72) Inventor: **ROBINSON, Thomas C.**
**100 Hill Road**
**Berkeley, CA 94708 (US)**
Inventor: **KITRILAKIS, Sotiris**
**33 Roble Road**
**Berkeley, CA 94705 (US)**
Inventor: **MARTIN, Jr., Thomas B.**
**6212 Rockwell Street**
**Oakland, CA 94618 (US)**

(74) Representative: **Patentanwälte Wenzel & Kalkoff
Ruhrstrasse 26 Postfach 2448
D-5810 Witten (DE)**

(56) References cited:
**Transactions A.S.A.I.O., Volume XIII, Issued
1977, pages 116-126, "Selection of Materials for
Ventricular Assist Pump Development and
Fabrication" by M. Szycher et. al. (See
Supplemental Sheet 2).**
**Transactions A.S.A.I.O., Volume XXIII Issued
1977, pages 519-525, "Long-Term in Vivo
Automatic Electronic Control of the Artificial
Heart" by D.L. Landis et al.**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

## Description

The invention relates to the field of cardiac prosthetic devices, and, more particularly to an implantable hydraulic action system, an implantable hydraulically actuated blood pumping system and to an implantable hydraulically actuated total cardiac prosthesis as defined in the respective introductory clauses of claims 1, 2 and 3.

It has been estimated that between 16,000 and 50,000 patients annually are suitable candidates for implantation of a total cardiac prosthesis (TCP). Such candidates typically are disabled due to insufficient left and right ventricular function but are otherwise in good health. Many thousands more annually with inadequate left ventricular function and satisfactory right ventricular function may be candidates for a permanently implanted left ventricular assist device (LVAD).

The ideal total cardiac prosthesis must provide complete rehabilitation for the patient. Such a TCP recipient must be able to engage in gainful employment and all normal activities including moderate exercise. He should retain a substantially normal appearance and normal or near normal mobility with no significant limitations of any kind. Cardiac output effected by the TCP must be normal, adequate and sufficiently responsive to the patient's requirements to accommodate expected, sudden changes in physical activity or emotional stress level. The presence and operation of the TCP must be sufficiently unobstrusive so that the patient can largely forget that he is dependent on an artificial heart. All blood pumping functions of the TCP should be completely automatic, so that the patient performs no control or monitoring functions except for maintaining adequate power to the TCP, and responding to warnings that indicate a lack of power or serious problems requiring immediate technical or medical attention.

The intrathoracic blood pumping components of the TCP must be similar in size and weight to the natural heart. TCP life must be sufficiently long and reliability sufficiently high that risk to the patient of sudden prosthesis failure and its attendant anxiety are minimized. The formation of pannus and adherent thrombus must be prevented to avoid a compromise of blood pump function. Thrombo-emboli and excessive blood damage also must be prevented. The TCP must not damage adjacent tissues or impair organ function by toxicity and adverse tissue reactions, by mechanical trauma or compression, or by excessive local temperatures. The system must avoid skin penetrations of any kind to prevent infections that can arise from percutaneous leads. This eliminates a major risk to the patient, reduces the need for clinical observation and treatment, and reduces the maintenance of the TCP required of the patient. This ideal system must be low in cost to purchase, implant, and maintain. The frequency and extent of routine monitoring and maintenance, both medical and technical, must be low.

Serious research toward the realization of a total cardiac prosthesis has been under way since about 1957, sponsored largely by the U.S. National Institutes of Health (NIH). Researchers have directed this activity to six principal areas: (1) blood-compatible materials for the blood pumping means; (2) heart valves; (3) blood pumps; (4) blood pump actuating means (5) power supplies and their application to the internal blood pump actuating means; and (6) control mechanisms for the pumping function.

Many materials have been developed which apparently achieve blood compatibility. See, e.g., the recent survey and evaluation of these by M. Szycher et al in "Selection of Materials for Ventricular Assist Pump Development and Fabrication," *Trans. ASAIO*, Vol. XXIII, p. 116, 1977, incorporated herein by reference. (As used herein, *Trans. ASAIO* refers to the *Transactions of the American Society of Artificial Internal Organs.*) While there is yet no human experience, recent materials like Biomer, Avcothane, etc. have been benign (i.e., have not caused thrombo-emboli) for periods up to 221 days in the calf. The materials for blood pumping membranes or sacs, however, must not only be benign and tissue compatible, but also able to withstand tens of pounds of force for something on the order of $10^9$ flexing cycles during a 20-year prosthesis life. Apparently, appropriate materials are nearly, if not already, realized today.

Another critical element of the blood pumping means is the valves, which permit blood flow into or out of the heart, but prevent backflow of blood. Many different types of valve prostheses have been developed and used in tens of thousands of implants to replace defective natural valves. Hence, adequate valves for a TCP appear to be well within the state of the art.

There has been a great deal of development activity in the area of blood pumps, primarily associated with LVADs. This experience has shown that by utilizing appropriate biocompatible materials as described above, adequate and reliable blood pumps can be designed. The most common form of blood pump is comprised of an elastomeric sac or diaphragm-capped cavity. In a TCP which comprises two such blood pumps, each cavity is fitted with an inlet valve and an outlet valve. These pumping cavities replicate the function of the adjacent right and left ventricles of the natural heart.

The least developed of the aforementioned areas of activity is the development of an actuator to couple the power supply to the blood pump. In order to squeeze the blood-pumping sacs or force the diaphragm into the blood pumping cavity, pneumatic actuation means supplied from outside the body are most common. A number of mechanical actuation systems may be found in the literature. All sorts of linkages, gears, cams, etc., have been proposed, but none is known to be successful. Most of these systems are driven by an electric motor, although some have relied upon piezoelectric devices and other esoteric means. Both copulsation, the technique used by the natural heart, and alternate pulsation of left and right ventricles have been employed successfully. See Smith, L. M., Olson, D. B., Sandquist, G., Grandall, E., Gentry, S., and Kolff, W. J., "A Totally Implantable Mechanical Heart," *Proceedings from the European Society of Artificial Organs*, Vol. 2, p. 150, 1975. Medical opinion appears to be impartial regarding this choice.

The coupling of a mechanical drive to the sensitive blood pumping diaphragm or sac is difficult to accomplish without raising excessive stresses and causing fatigue failures. Hence, the preferred coupling means is fluid, either liquid or gas. For example, one group has constructed an electric motor-powered, cam-actuated, diaphragm air pump which couples to the blood-pumping sac via pneumatic pressure. See V. Poirier et al, "Advances in Electrical Assist Devices," *Trans. ASAIO,* Vol. XXIII, p. 72, 1977, incorporated herein by reference. The entirety of the above-described mechanism is intended to be implanted within the thoracic cavity. In the Poirier et al design the motor rotates only once per heartbeat. Because relatively large torque is required from the motor, it must use strong magnetic fields, employ high current, and is rather heavy.

Burns et al, by contrast, constructed a TCP actuation system using a 10k—40k rpm motor driving a hydraulic pump pressurizing a liquid to actuate the blood-pumping bladders. See W. H. Burns et al, "The Totally Implantable Mechanical Heart, an Appraisal of Feasibility," *Annals of Surgery*, Sept. 1966, pp. 445—456, and W. H. Burns et al, "The Total Mechanical Cardiac Substitute," *Process in Cardiovascular Diseases,* Vol. XII, No. 3, 1969, pp. 302—311, both incorporated herein by reference. However, the electromechanically actuated hydraulic switching valve used in this and similar systems to shunt hydraulic fluid back and forth between ventricular actuating chambers has a number of disadvantages. The switching valve itself is relatively large and heavy, consumes a great deal of power and is potentially unreliable. Long and large ducts required in this type of system cause undesirable large frictional and inertial losses, and long fluid acceleration times.

Another approach to hydraulic actuation taken by researchers has involved the use of a reversible pump which directly pumps fluid back and forth between the two acutating chambers. See Jarvick U.S. Patent No. 4,173,796.

On the subject of power, up to this time most TCPs implanted in the calf have been powered pneumatically via transcutaneous tubing into the thoracic cavity. A large external console supplies the proper regimen of pressure variations in order to activate the internal blood pump. With such a system, calves have lived up to 221 days. Jarvik, "The Total Artificial Heart," *Scientific American,* Vol. 244, No. 1, pp. 74—80, January, 1981. On another tack, NIH has sponsored considerable effort on the development of internal nuclear power supplies and, to a lesser extent, of chemical fuel cells. None of this work, however, appears to be promising; in fact, the nuclear effort was terminated by the U.S. Energy Research and Development Administration. Additionally, various means of transmitting mechanical power transcutaneously have been attempted, but none appears to be promising. At present, transcutaneous transmission of electricity appears to be the preferred method for powering a TCP. A second, less preferable, possibility is the supplying of electrical power through percutaneous wire penetrations, but these always pose a threat of infection and are psychologically annoying to the patient.

Several investigators have developed the technique of transcutaneous electrical power transmission. Their approach is to implant a coil under the skin. This coil functions as a transformer secondary winding, receiving power from an inductively coupled, external, mating coil juxtaposed therewith to serve as the transformer primary winding. At frequencies on the order of 17 kHz up to 100 watts have been thus transmitted for many months across the skin of a dog, by Schuder. See, J. C. Schuder et al, "Ultra High Power Electromagnetic Energy Transport Into the Body," *Trans. ASAIO,* 1971, incorporated herein by reference. Thus, it appears that the inductive delivery across the intact skin of the approximately 30 watts needed to power a TCP is well within the state of the art.

On the subject of control of a TCP to make it sympathetic to the body, there have been many different approaches and much controversy. Some researchers have attempted to provide no active control. Others have required a control in order to achieve regular beating. See, e.g., W. H. Burns et al, "The Total Mechanical Cardiac Substitute," identified above. Some systems have attempted to control systole (i.e., the contraction phase of the cardiac cycle whose rate is one determinant of cardiac output) from the left ventricle of the TCP in order to control the systolic pressure in the aorta. Still other systems have attempted feedback control of stroke volume and beat rate.

The natural heart and at least some, if not all, TCPs are comprised of two pumps in series. The right pump receives blood from the vena cava and impels it into the pulmonary artery. The left pump receives blood from the pulmonary vein and impels blood into the main circulatory system via the aorta. These two pumps must, over time periods considerably longer than that of a few beats, pump nearly the same amount of blood. Otherwise,the delicate pulmonary circuit will either collapse or rupture from a deficiency or excess of blood pumped by the right ventricle relative to the left. Various investigators have included controls in their TCP systems in order to achieve the critical balance between the pumping rate of the right and left ventricles. The major intrinsic mechanism by which the natural heart controls cardiac output is described by Starling's Law, which essentially states that a ventricle will expel during systole essentially that blood which flows into the relaxed ventricle during diastole. For the right ventricle, the body controls the "tone," i.e., the pressure in the venous system, so that the pressure in the vena cava (relative to atmospheric pressure) may rise from 5 to 15 mm Hg when there is a demand for higher blood flow. This pressure change causes approximately a proportional increase in the amount of blood which flows from the vena cava through the tricuspid valve into the relaxed right ventricle during diastole.

It is important to note that the natural heart has no means to suck upon the veins. It can only produce a systolic contraction which expels blood from the ventricular chamber.

Similarly, for the left ventricle, the pressure in the pulmonary vein varies from 5 to 15 mm Hg and produces a proportional increase in blood flow into the left ventricle. If the right ventricle should temporarily pump slightly more than the left ventricle, the pressure rises in the pulmonary artery, and, as a consequence, in the pulmonary vein, causing more blood to flow into the left ventricle and thereby matching the pumping rate of the left ventrical to that of the right ventricle. Thus, the natural heart achieves the necessary balance between the two pumps in series via simple and direct fluid dynamic means. In a real sense, the heart is the servant, not the master of the circulatory system, and in particular it responds in the final analysis to the requirements of the body as reflected by the peripheral oxygen saturation. The above-described intrinsic control can maintain body function even in the absence of extrinsic humoral or neural control.

The body also neurally controls the rate at which the natural heart beats. Cardiac output is a function of the amount of blood ejected during systole, and the rate at which the heart beats. For all but the most strenuous activity, the systolic stroke volume per beat remains substantially constant. Thus, cardiac output is primarily a function of beat rate (i.e., the number of beats per minute). Heart rates can vary from a low of about 40 to as high as 220 beats per minute in a young person and ordinarily from about 60 to 150 bpm in an adult. Cardiac output of the natural heart can vary from about 4 to as high as 24 liters per minute, the latter being the case of a trained athlete. Experience with pacemakers and transplanted natural hearts shows that beat rate control via neural sensors is unnecessary for a satisfactory life. The hundreds of natural hearts which have been transplanted operate at their own beat frequency, unresponsive to the body's neural demands because there is no neural connection.

The natural control system also ensures that the systolic pressure in the aorta does not drop below about 80 mm Hg, in order to maintain adequate circulation to the brain. The mean pressure in the aorta is established by cardiac output and the peripheral resistance of the vascu-

lar systems. In some of the TCPs which previously have been developed, a control means has been provided to maintain pressure in the aorta and atrium within a reasonable range. On the other hand, there is evidence from natural heart transplants that such control is unnecessary; transplanted human hearts have no neural connections to the host body and hence their systolic rates are not related to neural control, yet people with such transplants have been able to lead meaningful lives. It may be concluded that a TCP can be satisfactorily operated without such control. The evidence above teaches that a workable TCP can be made to approximate the natural heart's Starling's Law behavior with relatively simple control operations.

Thus, a TCP is now technically feasible provided that a competent design is constructed. The critical blood pumping technology appears to be well established and adequate for long-term survival of the recipient. Benign power transmission across the skin can obviate the portent of infection of the thoracic cavity transmitted via percutaneous leads. One major area where satisfactory progress is lacking, however, is the provision of a practical blood pump actuating mechanism.

A typical actuating mechanism which represents the state of the art is disclosed in US-Specification 3,783,453. A flexible portion separates the blood from an actuation fluid which is pressurized by a pump and controlled by a valve. There is a sump from which the pump takes the actuating fluid and to which the control valve dumps the actuating fluid during the return stroke of the flexible portion. As already explained in connection with the actuation system of Burns et al the control valve is rather large and heavy, long and large ducts are required to connect the single recipients and chambers and there is the permanent threat of misfunction from the positively controlled switching operations.

Therefore, it is an object of this invention to improve an implantable hydraulic actuation system, a hydraulically actuated blood pumping system and a hydraulically actuated total cardiac prosthesis by reducing the length of the ducts to a necessary minimum, by enhancing reliability due to reduced switching and by minimizing the bulk of the prosthesis to a substantially unobtrusive device.

To meet this object the invention proposes an outlet of the actuation chamber for connecting this chamber to the fluid reservoir during the dump period and valve means for closing the outlet during the actuation period, the closing force of the valve means being generated by the fluid flow through said actuation fluid inlet path as far as an implantable hydraulic actuation system is concerned. For an implantable hydraulically actuated blood pumping system or total cardiac prosthesis the invention proposes an outlet of the actuation chamber for connecting this chamber to the fluid reservoir during the dump period and valve means for closing the outlet during the actuation period, the closing force of the valve means being generated by the fluid flow through said actuation fluid inlet path whereby said actuation chamber fills with actuation fluid and blood is expelled from said associated blood pumping chamber while said valve means is closed, and actuation fluid drains from said actuation chamber and said associated blood pumping chamber is allowed to fill with blood while said valve means is open.

The invention does not need an electrically actuated valve for connecting the actuation chamber with the actuation fluid reservoir during the dump period, because by the decreasing fluid flow at the end of a pulse the outlet of the actuation chamber is automatically connected to the actuation fluid reservoir due to the design of the valve means. The invention allows to define the actuation fluid reservoir at least in part by a compliant zone to which the outlet of the actuation chamber discharges directly. In this way a very compact, light and reliable prosthesis is achieved which allows the patient to engage in all normal activities without significant limitations of any kind.

Brief Description of the Drawings

The novel features of the invention are set out with particularity in the appended claims, but the invention will be understood more fully and clearly from the following detailed description of the invention as set forth in the accompanying drawings, in which:

Figure 1 is a block diagram showing the major components of a total cardiac prosthesis system according to the present invention;

Figure 2 is a sectional view of one type of blood pump suitable for use in a TCP according to the present invention;

Figure 3 is a schematic illustration of one embodiment of a total cardiac prosthesis actuation scheme according to the invention;

Figure 4 is a perspective view of one form of the TCP actuation scheme illustrated in Figure 3;

Figure 5 is a schematic illustration of another embodiment of a total cardiac prosthesis actuation scheme according to the invention;

Figure 6 is a schematic illustration of a portion of a TCP according to the invention showing one embodiment of a flow responsive ventricular dump valve for controlling the flow of actuation fluid out of each actuation chamber;

Figure 7 is a view similar to Figure 6 illustrating another embodiment of a flow responsive ventricular dump valve;

Figure 8 is a detail view of one form of the dump valve used in the embodiment of Figure 7;

Figure 9 is a perspective view, partly in section, of a TCP actuator housing having integral therein another embodiment of a flow responsive ventricular dump valve;

Figure 10 is a bottom plan view of the structure shown in Figure 9;

Figure 11 is a side elevational view of the structure shown in Figure 9;

Figure 12 is a sectional view of the structure shown in Figure 9 taken along line 12—12 of Figure 10, and showing the dump valve in its open position; and

Figure 13 is a view similar to Figure 12, but showing the dump valve in its closed position.

Detailed Description

The present invention is based at least in part on the discovery of a new approach to the controlled hydraulic actuation of a blood pump for use in cardiac prostheses. This actuation method and the various mechanical forms suitable for practicing it can be used with equal facility in total cardiac prostheses and circulatory assist devices. Since the similarities of structure and operation of these two classes of devices are well known in the art, the following description of this invention will relate primarily to the hydraulically actuated TCP.

The TCP System

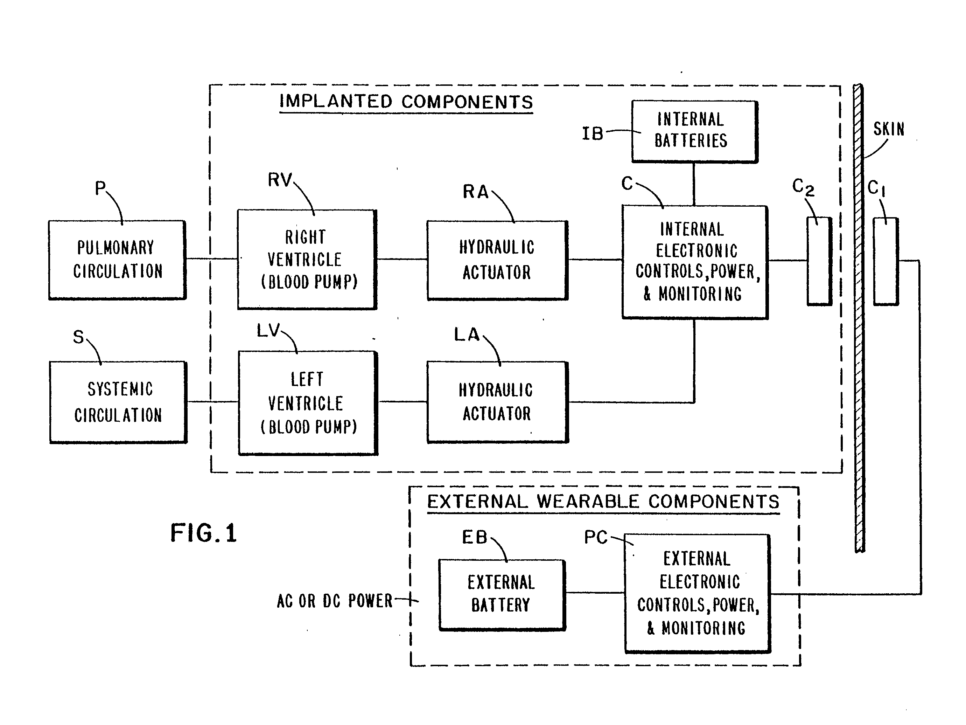

Figure 1 schematically illustrates the basic components of a TCP system according to the invention and the interaction of these components with the physiological systems of the patient. The pulmonary circulation P is maintained by a right ventricular blood pump RV. The systemic circulation S is maintained by a left ventricular blood pump LV. In the preferred embodiment of this invention each of the ventricular blood pumps is powered by a separate hydraulic actuator, RA for the right blood pump and LA for the left blood pump. The operation of the actuators RA and LA is controlled and monitored by an internal electronic control, power and monitoring circuit C which is powered at times by internal batteries IB. Most of the time, however, power is derived from an external power supply comprising an external battery EB and power circuit PC. External battery EB is rechargeable from a conventional power supply, such as household AC current or automotive DC current. Power is delivered transcutaneously to the implanted components by magnetic induction from a primary coil $C_1$ to a secondary coil $C_2$.

Preferably, the blood pumps and actuators are implanted within the thoracic cavity, while the internal electronic controls C and internal batteries IB are implanted outside of the thoracic cavity, preferably near the skin so as to permit easy replacement or servicing of these components by minor surgery. Of course, secondary coil $C_2$ must be located close to the skin for efficient inductive energy transfer.

Blood Pumps

The blood pumps of the TCP system according to this invention have essentially the same size, configuration and function as the natural heart. These functions include the same stroke volume capability, the same beat rate range, the same atrial filling pressure range and the same arterial pressure range and profile as in a healthy heart.

Blood pumps suitable for use according to the present invention can be of any of the known designs which are capable of being actuated by hydraulic actuation systems. This class includes systems in which the actuation fluid does work directly on a component of the blood pump as well as those systems in which the hydraulic fluid is coupled to the blood pump by indirect means, such as by magnetic coupling. Of primary interest, however, are those blood pump types in which the hydraulic fluid acts directly on a flexible portion of the blood pump. Examples of this type of blood pump include sac-type and membrane-capped cavity types generally known in the art. The preferred blood pumps for use in the TCP of this invention are of the membrane type (sometimes called bladder-type).

These preferred blood pumps essentially comprise a ventricular chamber containing blood inflow and outflow valves. The right and left ventricular blood pumps are generally of the same design except that the housings contain inflow and outflow ducts with orientations necessary to achieve appropriate implantability and fit.

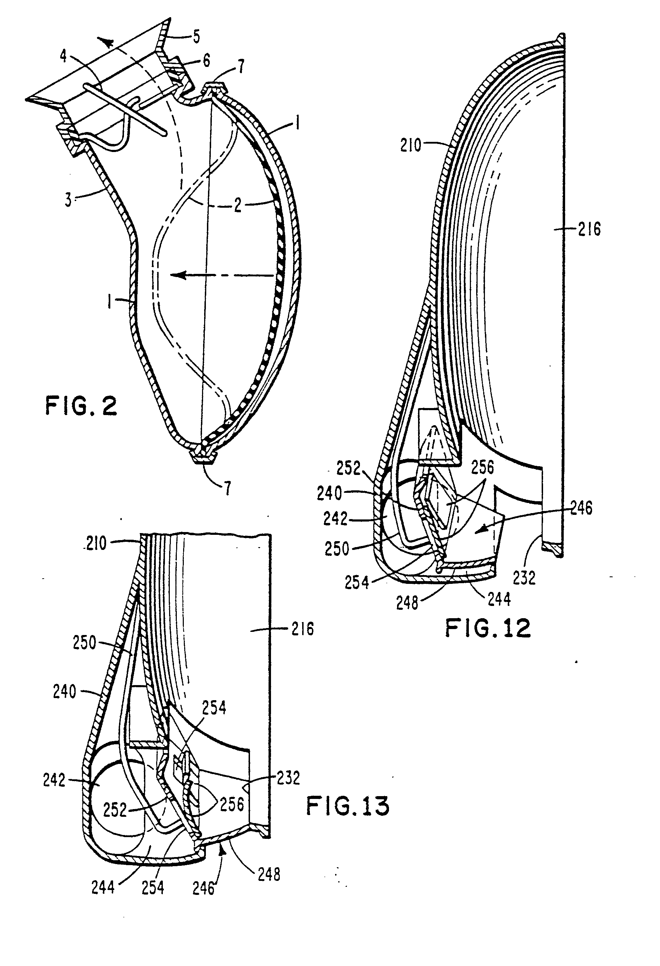

One preferred form of blood pump is schematically illustrated in Figure 2. The blood pump includes a two-piece rigid housing 1 in which is mounted a flexible membrane, or bladder 2, which is fabricated from an elastomeric material. The housing parts 1 and bladder 2 are secured together at their peripheries by a clamping ring 7. As the blood pump fills with blood during diastole, the flexible membrane assumes the position shown in Figure 2 in solid lines. As hydraulic fluid is added to the housing on the non-blood side of the membrane (in a manner later described), the change in hydraulic fluid volume causes displacement of the membrane to the position shown in dotted lines. As the membrane is displaced toward the opposite housing wall, blood is forcibly expelled from the blood pump. The membrane should be of such a design that the displacement or deformation occurs uniformly and consistently with each flexing stroke. The membrane also should be designed to intrinsically avoid blood damaging contact with any portion of the rigid housing, and/or extrinsic control means should be provided to so limit the excursion of the membrane.

The blood pumping chamber is provided with a blood outflow duct 3 containing a suitable prosthetic outflow valve device 4. An inflow duct (not shown) with a suitable prosthetic inflow valve also is provided. An example of suitable mechanical prosthetic valves are Bjork-Shiley valves although numerous other designs also may be employed. The blood pump inflow and outflow tracts preferably are connected respectively to known types of atrial cuffs 5 and arterial grafts (not shown) by snap-on quick-connect fittings 6 of any suitable design which facilitate surgical implantation of the TCP. The cuffs and grafts preferably are anastomosed to the atrial remanent and the aorta or pulmonary artery before the blood pumps are connected thereto.

The blood pumping member 2 preferably is of the single layer type formed from a high strength elastomeric biocompatible material. Polyurethane-based polymers such as Biomer and Avcothane are among the suitable materials for this application. These types of materials have been shown to exhibit high endurance and reliability in blood pumping operations. It is also important that the membrane of the blood pump exhibit low adhesion of thrombus and low generation of thrombo-emboli. The housing is formed of a suitable rigid metallic or plastic material, such as stainless steel coated with polyurethane or other biocompatible coatings, or glass or carbon fiber reinforced plastic. Typically, all internal surfaces of the blood pumps are coated with a suitable biocompatible material.

A suitable blood pump for use in the TCP of the present invention should be capable of providing a range of cardiac outputs of from 2.8 to about 9.5 liters per minute employing full stroke volume and at a beat rate of from about 35 to 120 beats per minute.

Actuation System

In the TCP of the present invention, the above-described blood pumps are hydraulically actuated. While any incompressible fluid which is compatible with the actuator system components can be employed, the preferred actuation fluid is physiologic saline solution (0.9 g percent NaCl) which is very close in saline composition to blood plasma. The use of saline as an actuation fluid promotes osmotic equilibrium and permits maintenance of a fixed actuation fluid inventory. It also eliminates the problems associated with the use of certain other actuation fluids such as silicone oils, including diffusion of these oils into the body or diffusion and mixing of body fluids in the actuation fluid which can cause degradation of the polymer materials in the flexible membrane.

The actuation system of the present invention in its most basic form comprises four basis components: (1) an actuation fluid reservoir or compliance sac, (2) actuation fluid pumping means, (3) a ventricular actuation chamber and (4) a flow responsive verticular dump valve. The basic manner of operation of this system involves the pumping of actuation fluid pulses from the actuation reservoir into the ventricular actuation chamber to displace the flexible blood pump membrane and expel blood from the pump. The ventricular dump valve, which operates in response to actuation fluid flow into the actuation chamber, serves both to close off the actuation chamber outlet during each fluid pulse and to drain or dump the actuation chamber fluid inventory at the end of each pulse, which permits refilling of the blood pumping chamber.

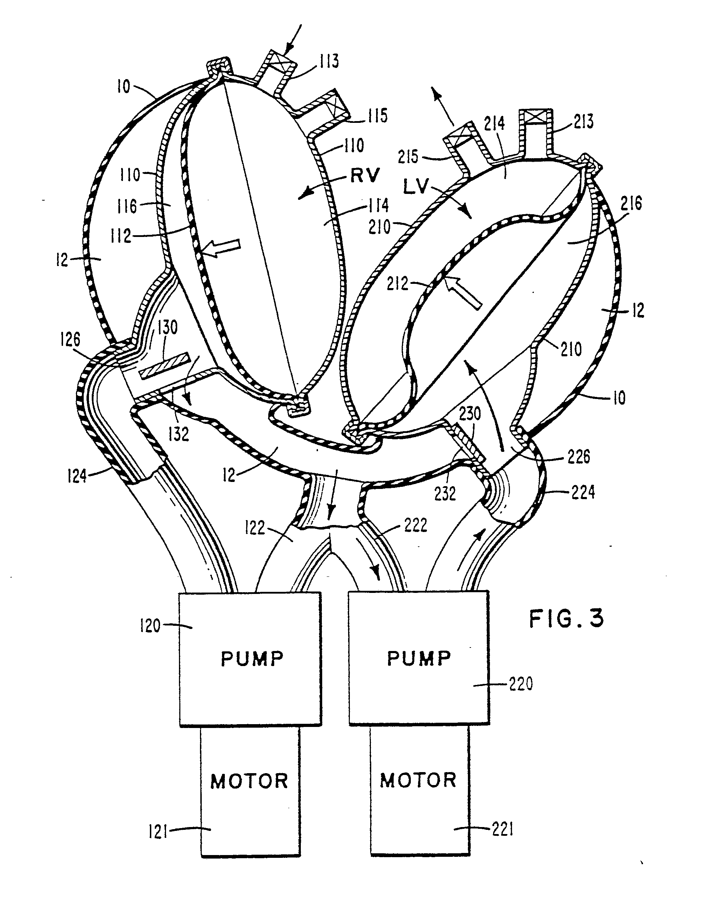

Figure 3 schematically illustrates a preferred actuation system for the TCP of the invention. The blood pumps include a right ventricle RV and a left ventricle LV. Right ventricle RV is defined by a rigid housing 110 which is divided by a flexible bladder 112 into a blood pumping chamber 114 and a fluid actuation chamber 116. Similarly, left ventricle LV is defined by a rigid housing 210 which is divided by a flexible bladder 212 into a blood pumping chamber 214 and a fluid actuation chamber 216. Valved blood inlets 113, 213 and valved blood outlets 115, 215 interconnect the blood pumping chambers 114, 214 with the appropriate blood vessels.

Portions of the blood pump housings 110 and 210 are surrounded by a flexible membrane 10 which defines a fluid containing reservoir or compliance sac 12. This compliance sac faces the lung and other soft tissues in the thoracic cavity and contains actuation fluid maintained at normal intrathoracic pressure levels. During operation, the fluid is dumped from each ventricle into the compliance sac during diastole and is removed from the compliance sac during systole. In the preferred form of operation, the ventricles are alternately actuated so as to minimize the change in volume of hydraulic actuation fluid in the system and therefore the overall size of the compliance sac. Copulsatile operation can, of course, be effected if desired.

In the preferred embodiment of Figure 3, each of the blood pumps is independently actuated by its own pumping means which preferably comprises a high speed, mixed flow, rotary pump driven by a brushless DC motor, although other suitable motor and pump designs may, of course, be used. The pump and motor bearings are totally immersed in and lubricated by the saline actuation fluid. In the preferred embodiment this hydraulic pump is designed to operate at a speed of about 7,000 to 15,000 rpm during the ventricular ejection phase (systole). To effect diastole the pump can be stopped, but it is preferred to provide a pump which can be slowed to about 1,000 to 1,200 rpm. At this low speed the pump will provide insufficient flow to actuate the flow responsive dump valve (described below), yet will maintain a full lubricating fluid film on the bearings.

Referring to Figure 3, actuation pump 120, driven by motor 121, draws fluid from compliance sac 12 through a flexible duct 122 and delivers it to actuation chamber 116 via a flexible duct 122 and delivers it to actuation chamber 116 via a flexible duct 124 through inlet 126. Similarly, actuating pump 220 driven by motor 221 draws fluid from compliance sac 12 through a flexible duct 222 and delivers it to actuation chamber 216 via flexible duct 224 through inlet 226. As indicated above, each hydraulic pump is started and operated during systole of its corresponding ventricle and stopped or slowed during diastole of that ventricle.

The actuation chamber is also provided with flow responsive ventricular dump valve means to effect dumping of hydraulic fluid from the actuation chamber at the end of systole. The term "flow responsive" is intended to include those valves which open and close the actuation chamber outlet primarily in response to forces which vary as a function of actuation fluid flow into the actuation chamber. Typically, this flow responsive mechanism will act in response to the pressure or momentum forces created by the incoming actuation fluid or to some combination of these forces. Such a flow actuated valve typically is provided with a threshold bias toward the open position. Accordingly, the valve closes only after the flow associated forces created during initial flow into the actuation chamber exceed this threshold. As long as the forces created by flow of actuation fluid into the actuation chamber are above this threshold, the valve will remain closed — i.e., throughout the systolic phase. When the flow of actuation fluid is stopped or reduced below the threshold bias forces, the bias returns the valve to its open position thereby dumping the actuation fluid from the chamber — i.e., the diastolic phase.

Referring again to the schematic representation in Figure 3, flow responsive valves 130, 230 are provided adjacent inlets 125, 226, respectively, for controlling the outflow of actuation fluid from actuating chambers 116, 216 into compliance sac 12 through outlets 132, 232. In the operational state shown in Figure 3, pump 220 is delivering actuator fluid to actuating chamber 216 through inlet 216. This inflow has caused valve 230 to close outlet 232, thereby preventing the escape of actuator fluid from actuating chamber 216. As pump 220 continues to operate, the volume of fluid within actuating chamber 216 increases, thereby compressing blood pumping chamber 214 and forcing blood outwardly therefrom through outlet 215 into the systemic vasculature. At the same time, pump 120 is not actively pumping actuation fluid to actuating chamber 116 through inlet 126. Hence, valve 130 remains in its open position, thereby allowing actuation fluid to drain from actuating chamber 116 into compliance sac 12. This drain of actuating fluid is caused by the right atrial blood pressure, which forces blood into the right ventricular blood pumping chamber 114 through inlet 113. Filling of each blood pumping chamber is therefore passive, as in the natural heart. When pump 120 is activated and pump 220 turned off or slowed down, the left ventricle is permitted to fill, while the right ventricle is compressed to eject blood into the pulmonary system through outlet 115. Of course, the pumps could be operated in copulsatile fashion, as long as a sufficient quantity of actuation fluid is present.

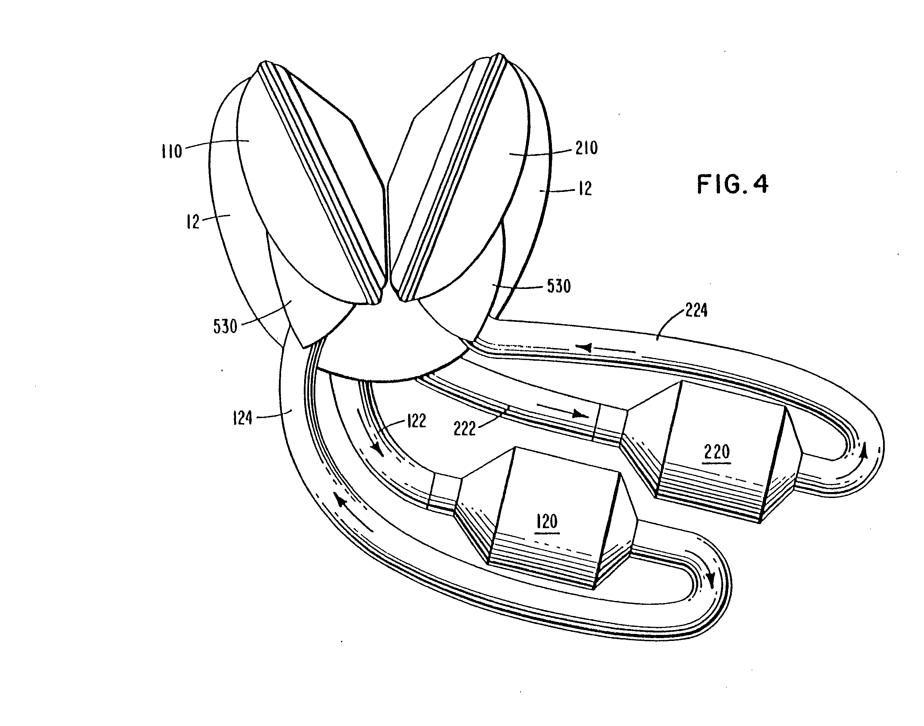

Figure 4 illustrates a preferred mechanical configuration for the actuation system embodiment shown schematically in Figure 3. Flow actuated dump valves 530 are integrally formed on ventricle housings 110, 210. Flexible conduits 122, 124, 222, 224 connect the pumps 120, 220 to dump valves 530 and the compliance sac 12.

Utilization of the above-described preferred embodiment provides a number of significant advantages. Utilization of two independent actuation systems allows for independent ventricular control as well as pump and motor optimization for each ventricle to maximize efficiency. Moreover, the use of flow actuated dump valves provides for the elimination of valve electromechanical actuators and thereby improves the reliability of the resulting TCP.

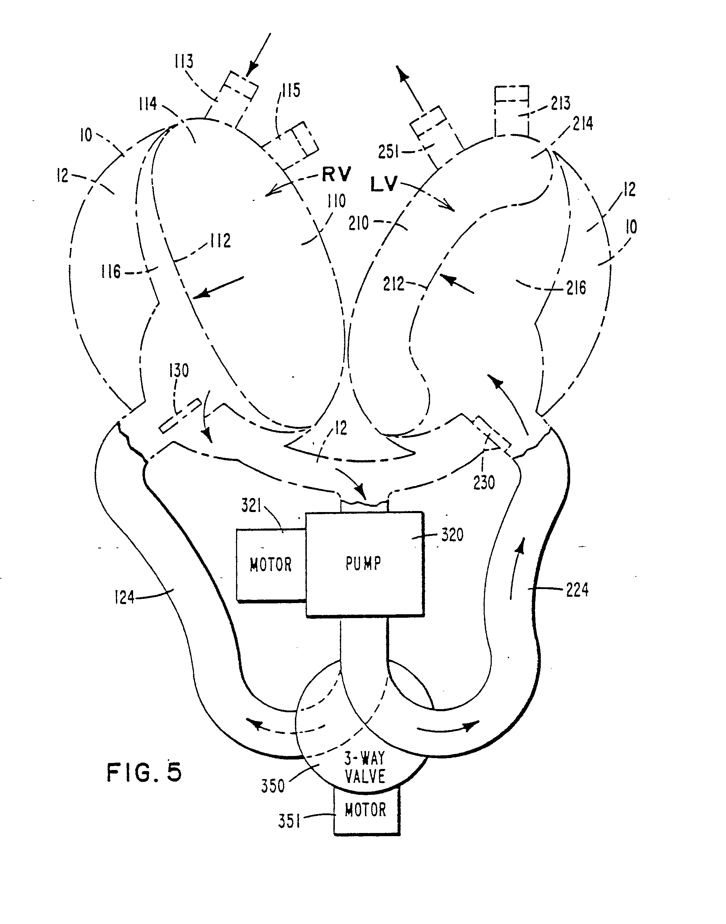

Figure 5 schematically illustrates an alternative embodiment in which the portion shown in dot-dash lines is identical to the corresponding portion of the embodiment shown in Figure 3. In this embodiment a single actuator pump 320 is driven by motor 321. An electromagnetically operated three-way switching valve 350, driven by motor 351, alternately diverts actuation fluid from the pump outlet to actuating chambers 116, 216 through flexible ducts 124, 224 in response to signals from the control circuit to provide alternate pulses of actuation fluid to the actuation chambers. If copulsatile operation is desired, valve 350 and motor 351 can be eliminated. Flow responsive dump valves 130, 230 used in this system are the same as those described generally in connection with Figure 3.

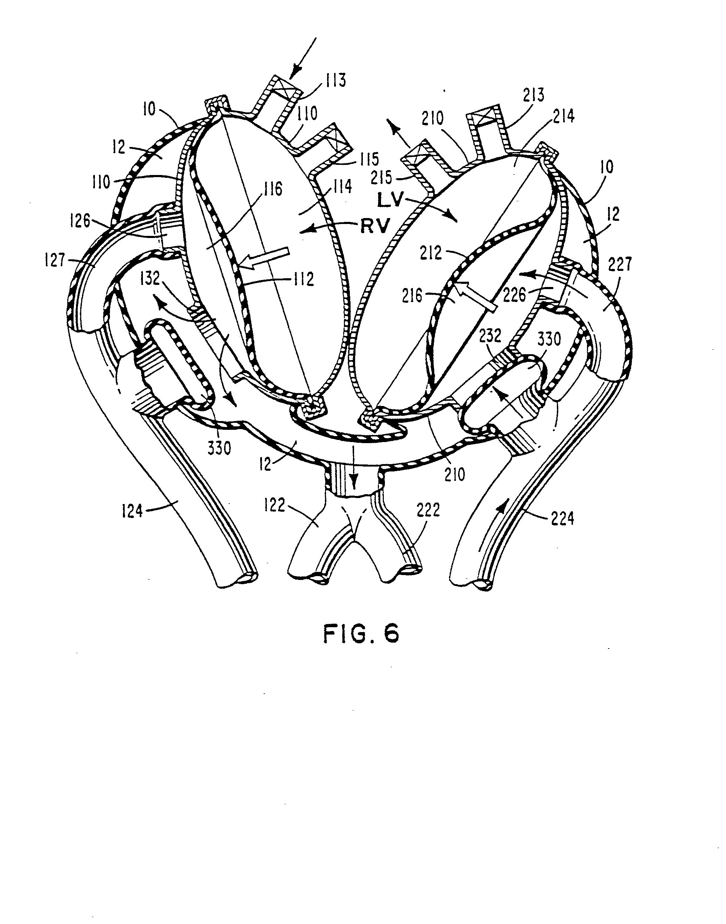

Figure 6 schematically illustrates one type of flow responsive dump valve useful according to the present invention. Each valve comprises an expandable mushroom shaped bladder 330. Bladders of other suitable shape may also be used. Bladder 330 may be made of an elastomeric material having a natural resiliency, or of a suitable flexible, non-elastic material. Bladders 330 expand against outlets 132, 232 of the actuation chambers when filled with pressurized actuation fluid delivered by actuator pumps 120, 220. Fluid inlets 126, 226 comprise branch conduits 127, 227. Outlet 232 is shown closed by valve 330, actuation fluid continuing past valve 330 into actuating chamber 216 through conduit 227. Outlet 132, on the other hand, is open. Either the natural resiliency of the elastomeric material, and/or a spring (not shown), may be employed to cause the bladder to return to the retracted (open) position.

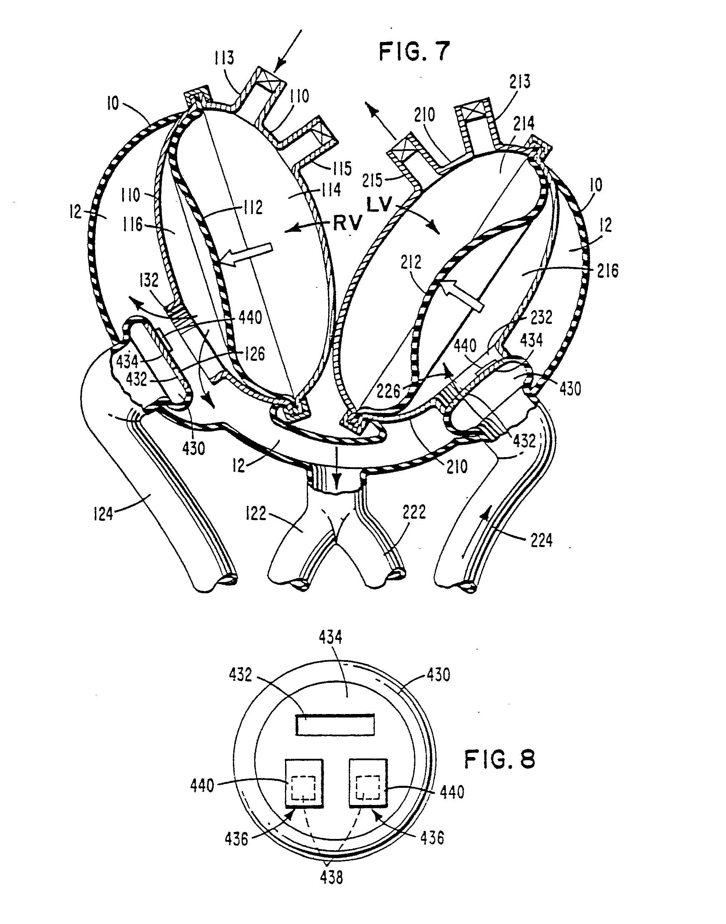

Figures 7 and 8 illustrate another embodiment of a flow responsive dump valve of the expandable bladder type used in both right and left ventricles. The valve will be described in connection with the left ventricle. The valve similarly comprises an expandable mushroom shaped bladder 430 which expands against outlet 232 of actuating chamber 216 when filled with actuation fluid delivered by actuator pump 220. Either the natural resiliency of the elastomeric material and/or a spring (not shown) may be employed to bias the bladder to its retracted (open) position.

In contrast to the embodiment of Figure 6, fluid inlet 226 comprises at least one orifice 432 in the cap of bladder 430. The cap comprises a substantially rigid wall portion 434 which can be formed of a suitable metallic or plastic material. In operation, when actuator pump 220 delivers actuating fluid to bladder 430, the fluid flows into actuation chamber 216 through orifice 432. Orifice 432 restricts the flow of actuation fluid sufficiently to cause the fluid pressure within bladder 430 to increase. When a sufficient pressure differential across cap 434 has developed to overcome the bladder's bias, the bladder is forced to expand against and close outlet 232, thereby preventing the escape of actuation fluid from actuation chamber 216. When the flow of actuation fluid into bladder 430 slows or stops, the pressure differential across cap 434 is reduced, and the bladder's bias causes it to retract to its open position, thereby opening outlet 232 and permitting actuation fluid to be dumped therefrom into compliance sac 12.

It is contemplated that a number of small orifices 432 can be employed, as long as they are sized to restrict fluid flow so that a sufficient pressure differential can develop quickly across cap 434 to expand bladder 430.

In order to optimize the efficiency of pump 220, a mechanism is provided whereby a substantially constant maximum pressure differential across cap 434 is maintained. This comprises a number of pressure-actuated valves 436 in cap 434. Each valve 436 may take the form of an aperture 438 covered by a leaf spring shutter 440 attached to cap 434 along one edge thereof. When the fluid pressure within bladder 430 exceeds a certain predetermined value, the spring bias of shutters 440 will be overcome and fluid will begin to flow into actuation chamber 216 through valves 436. The amount of fluid flowing through valves 436 is a function of the deflection of shutters 440. Shutters 440 will open to the extent necessary to maintain a substantially constant maximum pressure differential across cap 434. If desired, orifice 432 may be provided with a shutter which partially covers its opening.

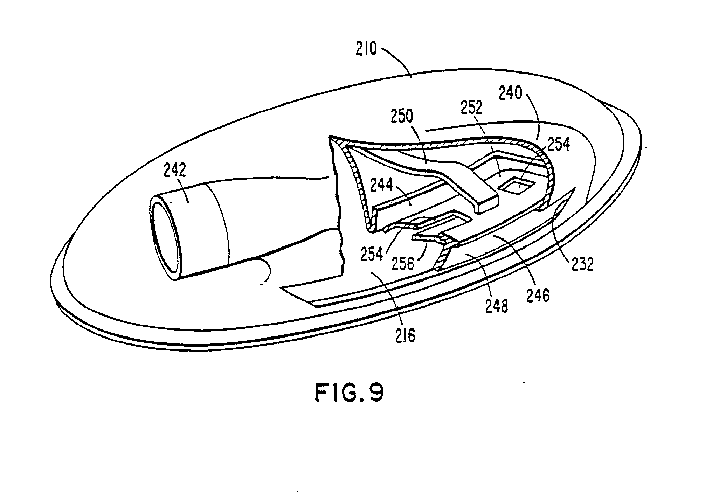

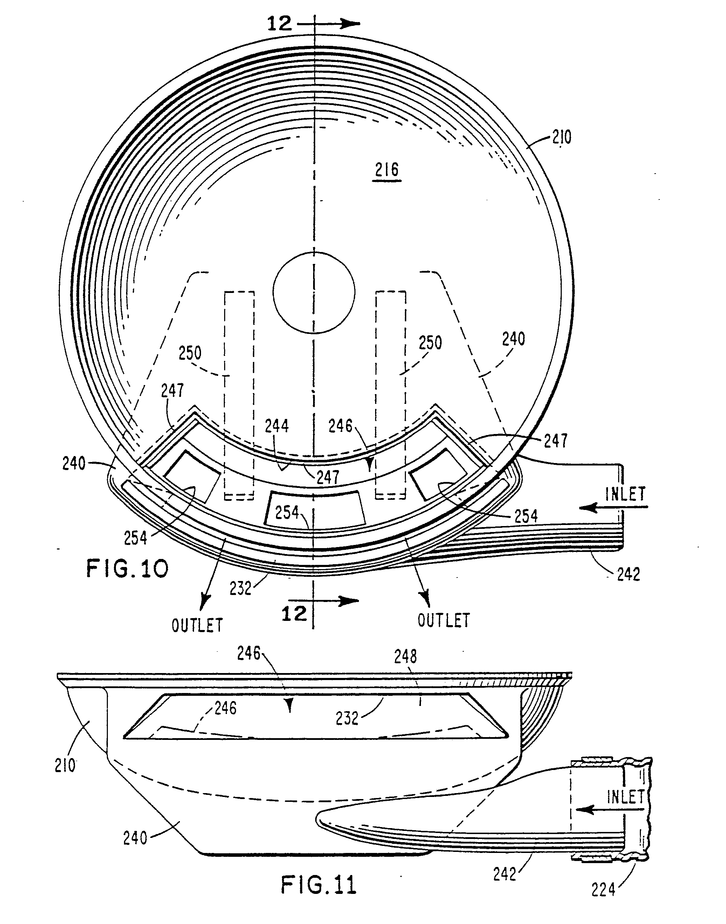

Figures 9 through 13 illustrate another embodiment of a flow responsive dump valve. The valve will be described in connection with the left ventrical. The figures illustrate the half of housing 210 which defines the actuating chamber 216. A manifold 240 is formed on housing 210. Actuator fluid is admitted into manifold 240 through an inlet nipple 242 which is adapted to be connected to flexible duct 224.

The interior of manifold 240 and the interior of actuating chamber 216 communicate by way of an elongated arcuate channel 244. An L-shaped arcuate valve piston 246 substantially fills the space within channel 244. A narrow slit 247 on the inner radius and sides of valve piston 246 remains for the fluid to flow from manifold 340 into actuating chamber 216. One leg 248 of piston 246 is adapted to cover and close arcuate outlet 232 formed in manifold 240 when the valve piston 246 moves downwardly as illustrated in Figure 9. Valve piston 246 is supported for arcuate movement within channel 244 on a pair of leaf springs 250 which are secured at one end to the interior of manifold 240 and at the other end to the top portion 252 of valve piston 246. Leaf springs 250 bias valve piston 246 in an upward direction, i.e., in a direction which will normally leave outlet 232 open. Valve piston 246 and leaf springs 250 are formed of a suitable metallic or plastic material, such as stainless steel or glass or carbon fiber reinforced plastic.

In operation, when actuator pump 220 delivers actuating fluid to manifold 240, the fluid flows into actuating chamber 216 through the narrow slit surrounding valve piston 246. When a sufficient pressure differential across valve piston 246 has developed to overcome the bias of leaf spring 250, valve piston 246 is forced downwardly to close outlet 232. When the flow of actuation fluid into manifold 240 slows or stops, the pressure differential across valve piston 246 is reduced, and the force of springs 250 causes valve piston 246 to move back to its open position, thereby opening outlet 232 and permitting actuation fluid to be dumped therefrom into compliance sac 12.

In order to optimize the efficiency of pump 220, a mechanism is provided whereby a constant pressure differential across valve piston 246 is maintained. This comprises a set of three apertures 254 in the top surface of valve piston 246. Apertures 254 are closed by leaf spring shutters 256 which flex downwardly to open apertures 254 for added fluid flow into actuating chamber 216 when the pressure of actuator fluid within manifold 240 exceeds a certain predetermined value. The greater the pressure within manifold 240, the greater the deflection of shutters 256, thereby maintaining the maximum pressure differential across valve piston 246 substantially constant.

The above-described valve can be designed to close at pressure drops across the piston which corresponds to minimum flow rates of from about 3 to 7 liters per minute. Typically, times for complete closure of this valve should be in the range of from about 10 to 50 milliseconds. The valve opening time is goverened by how quickly the volume of fluid swept by the piston can pass back through the slit into the actuation chamber. In valve closing the fluid can fill the actuation chamber of the ventricle while in valve opening it is assumed that fluid cannot flow back into the pump. Typical valve opening times (to full open position) on the order of from about 20 to 80 milliseconds are suitable for a valve of this design.

While the above description refers to a single dump valve associated with a single actuation chamber, it will, of course, be recognized that two or more dump valves of the same or different configurations can be associated with a single actuation chamber. While the above description refers generally to independently operable dump valves associated with each actuation chamber of a TCP, it is, of course, possible to utilize dump valves which operate in a mutually dependent manner by providing suitable coupling means.

Control and Operation

The above-described TCP system, especially in the preferred embodiments thereof, is particularly advantageous in its ability to respond to electronic controls which are designed to cause operation of the TCP in a manner consistent with natural heart operation in humans. Basically, a modified Frank-Starling mechanism is the sole means of blood pump response to the physiological needs of the implant recipient. Each blood pump ejects whatever blood fills it, and as a result, the atrial pressure is related to cardiac output in the manner similar to the Frank-Starling response of the normal heart. Under the Frank-Starling mechanism, cardiac output is equal to venous return. Since cardiac output is equal to the heart rate times stroke volume, changes in cardiac output can be achieved either by changing the heart rate or the stroke volume. It is preferred according to the present invention to keep the stroke volume constant and achieve changes in cardiac output by changing heart rate. Constant stroke volume may be achieved by intrinsic means, e.g., by use of a stroke-limiting diaphragm, or by providing extrinsic control of diaphragm excursions in a known manner.

In the preferred embodiment of Figure 3, heart rate control is effected by delivering controlled intermittent pulses of actuation fluid to the actuation chamber in response to a control signal supplied to each actuation fluid pump motor by control circuit C (Fig. 1). This control signal starts and stops (or speeds up and slows down) the pumps to cause the ventricular dump valves to close and open in the manner described above at the beginning and end of systole. This control signal can be generated in response to any of a number of known measurable variables which provide information that can be employed to effect blood pump operation to ensure that the physiologic requirements of blood flow are met. One such variable is atrial pressure, which can be measured in a known manner using pressure transducers.

In the embodiment of Figure 5, alternately pulsatile operation can be controlled by supplying a control signal to the motor operated switching valve which governs the destination and duration of each fluid pulse.

In the preferred manner of operating the TCP of the present invention, ventricular ejections will alternate thereby conserving actuation fluid. As indicated previously, however, copulsatile operation can be effected with a concomitant increase in the hydraulic reservoir capacity. One of the significant advantages of the preferred TCP design of the present invention is that by the use of separate actuation mechanisms for each ventricle, separate and optimal control of each ventricle can be achieved.

Power Supply

In the preferred embodiment power to energize the electric motors and control electronics is furnished by electromagnetic induction across the intact skin of the patient. Telemetry signals for indicating system and patient information are also transmitted in this manner, albeit usually in the opposite direction. This type of power supply and telemetering system has been described in the prior art and consists generally of a high frequency coupling transformer which includes a small flat internal coil implanted subcutaneously and a larger flat external coil which is mounted over the implanted coil. The external coil can be carried in a vest, belt or other article of clothing. In practice, this energy transmission system can tolerate considerable movement of the external coil relative to the internal coil without adversely affecting the transfer of power or information into or out of the system. Power to drive the external coil can be provided in an external electronics pack which can contain batteries, battery charging electronics and other electronic systems useful in the monitoring of system and patient functions. Also included in the monitoring electronics are provisions for audible or visible alarms which warn of incipient malfunction or problems.

The external battery pack can be designed to provide several hours of mobility for the patient. This battery pack when depleted can be easily replaced with a fully charged one. It can also be charged from AC line current when the patient is "plugged into the wall" or while the patient is using another battery pack, or from an automotive DC battery while motoring.

An implanted internal battery pack will also provide a temporary source with complete freedom from any external power. This will permit the recipient to undertake acts such as bathing and the like and will provide sufficient time to change external power sources, i.e., changing the vest or external battery packs. In the preferred embodiment this internal battery will be located subcutaneously for easier periodic replacement.

While certain specific embodiments of the invention have been described with particularity herein, it should be recognized that various modifications thereof will appear to those skilled in the art. Therefore, the scope of the invention is to be limited solely by the scope of the claims appended hereto.

## Claims

1. An implantable hydraulic actuation system for supplying motive power to a blood pumping chamber (114, 214) having a flexible portion (112, 212) comprising: an actuation fluid reservoir (12); actuation fluid pumping means (120, 220, 320) in fluid communication with said reservoir for providing intermittent pulses of actuation fluid; and an actuation chamber (116, 216) having an actuation fluid inlet path (124, 224) in fluid communication with said pumping means, said actuation chamber being adapted to cause displacement of the flexible portion of said blood pumping chamber in response to changes in volume of actuation fluid in said actuation chamber, characterized by an outlet (132, 232) of the actuation chamber (116, 216) for connecting this chamber to the fluid reservoir (12) during the dump period and by valve means (130, 230, 330, 430, 446) for closing the outlet during the actuation period, the closing force of the valve means being generated by the fluid flow through said actuation fluid inlet path.

2. An implantable hydraulically actuated blood pumping system comprising a blood pumping chamber (114, 214) having a blood inlet (113, 213), a blood outlet (115, 215) and a flexible portion (112, 212); an actuation fluid reservoir (12); actuation fluid pump means (120, 220, 320) in fluid communication with said reservoir for providing intermittent pulses of actuation fluid; and an actuation chamber (116, 216) having an actuation fluid inlet path (124, 224) in fluid communication with said pumping means, said actuation chamber being operatively associated with said blood pumping chamber to cause displacement of said flexible portion of said blood pumping chamber in response to changes in volume of actuation fluid in said actuation chamber, characterized by an outlet (132, 232) of the actuation chamber (116, 216) for connecting this chamber to the fluid reservoir (12) during the dump period and by valve means (130, 230, 330, 430, 446) for closing the outlet during the actuation period, the closing force of the valve means being generated by the fluid flow through said actuation fluid inlet path whereby said actuation chamber fills with actuation fluid and blood is expelled from said associated blood pumping chamber (114, 214) while said valve means is closed, and actuation fluid drains from said actuation chamber and said associated blood pumping chamber is allowed to fill with blood while said valve means is open.

3. An implantable hydraulically actuated total cardiac prosthesis comprising a pair of blood pumping chambers (114, 214) each having a blood inlet (113, 213), a blood outlet (115, 215) and a flexible portion (112, 212); a pair of actuation chambers (116, 216) each having an actuation fluid inlet path (124, 224), one of said actuation chambers being operatively associated with one of said blood pumping chambers and the other of said actuation chambers being operatively associated with the other of said blood pumping chambers to cause displacement of the flexible portion of said blood pumping chambers in response to changes in volume of actuation fluid in their associated actuation chambers; an actuation fluid reservoir (12) and actuation fluid pumping means (120, 220) in fluid communication with said reservoir (12) and said actuation fluid inlet paths (124, 224) for providing intermittent pulses of actuation fluid from said resevoir to said actuation chambers, characterized by an outlet (132, 232) of the actuation chamber (116, 216) for connecting this chamber to the fluid reservoir (12) during the dump period and by valve means (130, 230, 330, 430, 446) for closing the outlet during the actuation period, the closing force of the valve means being generated by the fluid flow through said actuation fluid inlet path whereby said actuation chamber fills with actuation fluid and blood is expelled from said associated blood pumping chamber (114, 214) while said valve means is closed, and actuation fluid drains from said actuation chamber and said associated blood pumping chamber is allowed to fill with blood while said valve means is open.

4. The invention of claim 1, 2 or 3 wherein said actuation fluid reservoir (12) comprises a compliant zone defined at least in part by a flexible wall portion (112, 212) made from bio-compatible elastomeric material.

5. The invention of claim 4 wherein said actuation fluid reservoir (12) also includes flexible ducting (122, 222) leading to and from said actuation fluid pumping means (120, 220, 320) and to and from said actuation chamber (116, 216).

6. The invention of claim 3 wherein said actuation fluid reservoir (12) comprises a separate compliant zone associated with each of said actuation chambers (116, 216), defined at least in part by a flexible wall portion (10) made from biocompatible elastomeric material, said compliant zones communicating with each other, said pumping means (120, 220, 320) and said actuation chambers (116, 216) by flexible ducting (124, 224, 122, 222).

7. The inventions of claim 1 or 2 wherein said actuation fluid pumping means comprises a pump (120, 220, 320) and drive motor (121, 221, 321) therefor.

8. The invention of claim 7 wherein said pump (121, 221, 321) is a high speed, mixed flow rotary pump.

9. The invention of claim 7 wherein said drive motor (121, 221, 321) is a brushless DC electric motor.

10. The invention of claim 9 further comprising means for commutating said brushless DC electric motor.

11. The invention of claim 10 wherein said means for commutating comprises back-emf control circuitry.   .

12. The invention of claim 3 wherein said actuation - fluid pumping means comprises a separate pump (120, 220) and drive motor (121, 221) therefor associated with each of said actuation chambers (116, 216).

13. The invention of claim 12 wherein each of said pumps (120, 220) is a high speed, mixed flow rotary pump.

14. The invention of claim 12 wherein each of said drive motors (121, 221) is a brushless DC electric motor.

15. The invention of claim 14 further comprising means for commutating said brushless DC electric motor.

16. The invention of claim 15 wherein said means for commutating comprises back-emf control circuitry.

17. The invention of claim 3 wherein said actuation fluid pumping means comprises a pump (320) and drive motor (321) therefor and a three-way switching valve (350) associated with the pump outlet for alternately directing the pump output to one or the other of said actuation chambers (116, 216).

18. The invention of claim 17 wherein said pump (320) is a high speed, mixed flow rotary pump.

19. The invention of claim 17 wherein said drive motor (321) is a brushless DC electric motor.

20. The invention of claim 19 further comprising means for commutating said brushless DC electric motor.

21. The invention of claim 20 wherein said means for commutating comprises back-emf control circuitry.

22. The invention of claim 17 wherein said three-way switching valve (350) has a separate drive motor (351) associated therewith.

23. The invention of claim 2 or 3 wherein said blood inlet (113, 213) and said blood outlet (115, 215) are provided with prosthetic valves adapted to permit the flow of blood in only one direction.

24. The invention of claim 2 or 3 wherein said blood pumping chamber (114, 214) comprises a variable volume zone defined in part by a flexible membrane (112, 212) made from biocompatible elastomeric material and in part by a rigid blood pumping chamber housing (110, 210).

25. The invention of claim 1, 2 or 3 wherein said actuation chamber (116, 216) comprises a variable volume zone defined in part by said flexible portion (112, 212) of said blood pumping chamber (114, 214) and in part by a rigid actuation chamber housing (110, 210).

26. The invention of claim 1, 2 or 3 wherein said valve means is adapted:

to close said actuation fluid outlet path (132, 232) at the beginning of each of said fluid pulses when sufficient flow of actuation fluid is supplied to said actuation fluid inlet path (124, 224) to create forces above a predetermined threshold magnitude;

to keep said actuation fluid outlet path (132, 232) closed during said pulses while said flow through said inlet path (124, 224) is sufficient to maintain said forces above said predetermined threshold magnitude; and

to open said actuation fluid outlet path (132, 232) at the end of said pulses when said flow through said inlet path (124, 224) is reduced to a value at which said forces fall below said predetermined threshold magnitude.

27. The invention of claim 26 wherein said valve means includes spring means (250) for exerting a permanent bias force tending to open said actuation fluid outlet path (132, 232), and the magnitude of said bias force is equal to said predetermined threshold.

28. The invention of claim 27 wherein said valve means is adapted to act in response to pressure forces created by the flow of actuation fluid through said inlet path (124, 224, 242).

29. The invention of claim 28 wherein said valve means comprises a hollow closure member (330, 430) having an interior in fluid communication with actuation fluid delivered by said pumping means, and movable between a closed position blocking said actuation fluid outlet path (132, 232) when filled with actuation fluid during fluid pulses, and an open position clear of said actuation fluid outlet path (132, 232) between fluid pulses.

30. The invention of claim 29 wherein said closure member (330, 430) comprises an expandable envelope.

31. The invention of claim 30 wherein said envelope comprises elastomeric material.

32. The invention of claim 27 wherein said valve means comprises a closure member (430, 246) movable between a closed position blocking said fluid outlet path (132, 232) during fluid pulses, and an open position clear of said actuation fluid outlet (132, 232) between fluid pulses.

33. The invention of claim 32 wherein said valve means is adapted to act in response to differential pressure forces.

34. The invention of claim 33 wherein said fluid inlet path includes at least one orifice (432, 254) through said closure member (430, 246) through which actuation fluid flows from said pumping means into said actuation chamber (116, 216).

35. The invention of claim 34 wherein said closure member (430) is hollow and has an interior in fluid communication with actuation fluid delivered by said pumping means, and said orifice (432) is formed through the wall of said hollow closure member.

36. The invention of claim 35 wherein said closure member (430) comprises an expandable envelope.

37. The invention of claim 36 wherein said orifice is formed through a substantially rigid wall portion of said envelope.

38. The invention of claim 37 wherein said envelope comprises elastomeric material.

39. The invention of claim 36 wherein said envelope comprises elastomeric material.

40. The invention of claim 34 further comprising means (440, 256) for maintaining a substantially constant pressure differential across said closure member (430, 246) during fluid pulses despite changes in flow rate of fluid along said fluid inlet path.

41. The invention of claim 40 wherein said means for maintaining a substantially constant pressure differential comprises at least one aperture (254) through said closure member, and shutter means (440, 256) biased to substantially close said aperture, but movable to open said aperture to an extent proportional to the flow rate of fluid along said fluid inlet path.

42. The invention of claim 41 wherein said shutter means comprises a leaf spring shutter (256) overlying said aperture (254).

43. The invention of claim 41 wherein said closure member (430) is hollow and has an interior in fluid communication with actuation fluid delivered by said pumping means, and said orifice (432) and said aperture are formed through the wall of said hollow closure member (430).

44. The invention of claim 43 wherein said closure member (430) comprises an expandable envelope.

45. The invention of claim 44 wherein said orifice (432) and said aperture are formed through a substantially rigid wall portion (434) of said envelope.

46. The invention of claim 45 wherein said envelope comprises elastomeric material.

47. The invention of claim 44 wherein said envelope comprises elastomeric material.

48. The invention of claim 2 or 3 further comprising means ($C_1$, $C_2$) for applying electric power to said actuation fluid pumping means and associated control electronics (C) in a patient having an implanted cardiac prosthesis.

49. The invention of claim 48 wherein said means for supplying electric power comprises an external primary coil ($C_1$) inductively coupled to a subcutaneous secondary coil ($C_2$) for transmitting electrical energy across the intact skin of said patient.

50. The invention of claim 33 wherein said actuation chamber (216) includes a manifold (240) and a flow gate (244) intercommunicating the interior of said manifold (240) and the interior of said actuation chamber (216) through which actuation fluid passes when filling said actuation chamber (216), said actuation fluid inlet and fluid outlet paths being formed in the wall of said manifold, and said closure member (246) is disposed within and partially blocks said flow gate (244), and is movable within said flow gate to its closed position at the beginning of each fluid pulse when the differential pressure force across said closure member exceeds the magnitude of the force of said spring means (250).

51. The invention of claim 50 wherein said closure member (246) is hinged and undergoes pivotal movement between said closed and open positions.

52. The invention of claim 51 wherein said spring means comprises a leaf spring (250) secured at one end to the wall of said manifold (240) and hingedly supporting said closure member (246) at its other end.

53. The invention of claim 51 wherein said actuation fluid outlet path (232) is disposed at one side of and transverse to said flow gate (244), and said closure member comprises a shutter (246) movable across said actuation fluid outlet (232) and a swing arm (250) connected at one end to said shutter (246), extending across said flow gate and secured at its opposite end to the wall of said manifold (240) on the opposite side of said flow gate (244).

54. The invention of claim 53 wherein said swing arm (250) is said spring means and comprises a leaf spring hingedly supporting said shutter (246).

55. The invention of claim 54 wherein said closure member (246) is provided with means for maintaining a substantially constant pressure drop across said closure member (246) despite changes in flow rate of fluid into said manifold (240).

56. The invention of claim 55 wherein said means for maintaining a relatively constant pressure drop comprises a leaf spring shutter (256) biased to substantially close said orifice (254) but movable to open said orifice to an extent proportional to the flow rate of fluid into said manifold (240).

## Patentansprüche

1. Einpflanzbares hydraulisches Aktivierungssystem zur Versorgung einer Blutpumpenkammer (114, 214), die einen flexiblen Bereich (112, 212) aufweist, mit einer Bewegungskraft, bestehend aus: einem Aktivierungsflüssigkeitsbehälter (12); Pulmpmitteln (120, 220, 320) für die Aktivierungsflüssigkeit, die mit dem Behälter zur Erzeugung intermittierender Impulse der Aktivierungsflüssigkeit flüssigkeitsleitend verbunden sind; und aus einer Aktivierungskammer (116, 216), die einen mit den Pumpmitteln in flüssigkeitleitender Verbindung stehenden Aktivierungsflüssigkeitseinlaß (124, 224) aufweist, wobei die Aktivierungskammer eine Bewegung des flexiblen Bereichs der Blutpumpenkammer in Abhängigkeit der Volumenänderung der Aktivierungsflüssigkeit in der Aktivierungskammer bewirkt, gekennzeichnet durch einen Auslaß (132, 232) der Aktivierungskammer (116, 216) zur Verbindung dieser Kammer mit dem Flüssigkeitsbehälter (12) während der Leerperiode und durch Ventilmittel (130, 230, 330, 430, 446) zum Schließen des Auslasses während der Aktivierungsperiode, wobei die Schließkraft der Ventilmittel von dem Flüssigkeitsstrom durch den Aktivierungsflüssigkeitseinlaß aufgebracht wird.

2. Einpflanzbares hydraulisch aktiviertes Blutpumpensystem bestehend aus einer Blutpumpenkammer (114, 214) mit einem Bluteinlaß (113, 213), einem Blutauslaß (115, 215) und einem flexiblen Bereich (112, 212); einem Aktivierungsflüssigkeitsbehälter (12); Pumpmitteln (120, 220, 320) für die Aktivierungsflüssigkeit, die zur Erzeugung intermittierender Impulse der Aktivierungsflüssigkeit in flüssigkeitsleitender Verbindung mit dem Behälter stehen; und aus einer Aktivierungskammer (116, 216) mit einem Aktivierungsflüssigkeitseinlaß (124, 224), der in flüssigkeitsleitender Verbindung mit den Pumpmitteln steht, wobei die Aktivierungskammer mit der Blutpumpenkammer in Wirkverbindung steht, um eine Bewegung des flexiblen Abschnitts der Blutpumpenkammer in Abhängigkeit der Volumenänderung der Aktivierungsflüssigkeit in der Aktivierungskammer zu bewirken, gekennzeichnet durch einen Auslaß (132, 232) der Aktivierungskammer (116, 216) zur Verbindung dieser Kammer mit dem Flüssigkeitsbehälter (12) während der Leerperiode und durch Ventilmittel (130, 230, 330, 430, 446) zum Schließen des Auslasses während der Aktivierungsperiode, wobei die Schließkraft der Ventilmittel infolge des Flüssigkeitsstromes durch den Aktivierungsflüssigkeitseinlaß bewirkt wird, wodurch sich die Aktivierungskammer mit Aktivierungsflüssigkeit füllt und Blut aus der wirkverbundenen Blutpumpenkammer (114, 214) gedrückt wird, während die Ventilmittel geschlossen sind, und wodurch Aktivierungsflüssigkeit aus der Aktivierungskammer ausfließt und sich die wirkverbundene Blutpumpenkammer mit Blut füllen kann, während die Ventilmittel geöffnet sind.

3. Einpflanzbare hydraulisch aktivierte Herz-Totalprothese bestehend aus einem Paar von Blutpumpenkammern (114, 214) mit jeweils einem Bluteinlaß (113, 213), einem Blutauslaß (115, 215) und einem flexiblen Bereich (112, 212); einem Paar von Aktivierungskammern (116, 216) mit jeweils einem Aktivierungsflüssigkeitseinlaß (124, 224), wobei eine der Aktivierungskammern in Wirkverbindung mit einer der Blutpumpenkammern und die andere Aktivierungskammer in Wirkverbindung mit der anderen Blutpumpenkammer steht, um eine Bewegung des flexiblen Bereiches jeder Blutpumpenkammer in Abhängigkeit der Volumenänderung der Aktivierungsflüssigkeit in den wirkverbundenen Betätigungskammern hervorzurufen; einem Aktivierungsflüssigkeitsbehälter (12); und aus Aktivierungsflüssigkeitspumpmitteln (120, 220), die in flüssigkeitsleitender Verbindung mit dem Behälter (12) und den Aktivierungsflüssigkeitseinlässen (124, 224) stehen, um intermittierende Impulse der Betätigungsflüssigkeit aus dem Behälter zu den Aktivierungskammern bereitzustellen, gekennzeichnet durch einen Auslaß (132, 232) der Betätigungskammer (116, 216) zur Verbindung dieser Kammer mit dem Flüssigkeitsbehälter (12) während der Leerperiode und durch Ventilmittel (130, 230, 330, 430, 446) zum Schließen des Auslasses während der Betätigungsperiode, wobei die Schließkraft der Ventilmittel infolge des Flüssigkeitsstromes durch den Aktivierungsflüssigkeitseinlaß bewirkt wird, wodurch sich die Aktivierungskammer mit Aktivierungsflüssigkeit füllt und Blut aus der wirkverbundenen Blutpumpenkammer (114, 214) gedrückt wird, während die Ventilmittel geschlossen sind, und wodurch Aktivierungsflüssigkeit aus der Aktivierungskammer ausfließt und sich die wirkverbundene Blutpumpenkammer mit Blut füllen kann, während die Ventilmittel geöffnet sind.

4. Erfindung nach Anspruch 1, 2 oder 3, bei der der Aktivierungsflüssigkeitsbehälter (12) eine nachgiebige Zone umfaßt, die zumindest teilweise durch einen flexiblen Wandbereich (112, 212) aus einem bio-kompatiblen, elastomerischen Material festgelegt ist.

5. Erfindung nach Anspruch 4, bei der der Aktivierungsflüssigkeitsbehälter (12) außerdem eine flexible Leitung (122, 222) umfaßt, die zu und von den Aktivierungsflüssigkeitspumpmitteln (120, 220, 320) und zu und von der Aktivierungskammer (116, 216) führt.

6. Erfindung nach Anspruch 3, bei der der Aktivierungsflüssigkeitsbehälter (12) eine gesonderte nachgiebige Zone umfaßt, die mit jeder der Aktivierungskammern (116, 216) verbunden ist und zumindest teilweise durch einen flexiblen Wandbereich (10) aus einem bio-kompatiblen, elastomerischen Material festgelegt ist, wobei die nachgiebigen Zonen miteinander, mit den Pumpmitteln (120, 220, 320) und den Aktivierungskammern (116, 216) mit Hilfe von flexiblen Leitungen (124, 224, 122, 222) kommunizieren.

7. Erfindung nach Anspruch 1 oder 2, bei der die Aktivierungsflüssigkeitspumpmittel eine Pumpe (120, 220, 320) und einen dafür vorgesehenen Antriebsmotor (121, 221, 321) umfassen.

8. Erfindung nach Anspruch 7, bei der die Pumpe (121, 221, 321) eine Hochgeschwindigkeits-Mischstrom-Rotorpumpe ist.

9. Erfindung nach Anspruch 7, bei der der Antriebsmotor (121, 221, 321) ein bürstenloser Gleichstrom-Elektromotor ist.

10. Erfindung nach Anspruch 9, die außerdem Mittel zu Umsteuerung des bürstenlosen Gleichstrom-Elektromotors enthält.

11. Erfindung nach Anspruch 10, bei der die Mittel zur Umsteuerung eine Gegen-EMK-Steuerschaltung umfassen.

12. Erfindung nach Anspruch 3, bei der die Aktivierungsflüssigkeitspumpmittel für jede der Aktivierungskammern (116, 216) eine gesonderte Pumpe (120, 220) und einen dafür vorgesehenen Antriebsmotor (121, 221) umfassen.

13. Erfindung nach Anspruch 12, bei der jede der Pumpen (120, 220) eine Hochgeschwindigkeits-Mischstrom-Rotorpumpe ist.

14. Erfindung nach Anspruch 12, bei der jeder der Antriebsmotoren (121, 221) ein bürstenloser Gleichstrom-Elektromotor ist.

15. Erfindung nach Anspruch 14, bei der außerdem Mittel zur Umsteuerung des bürstenlosen Gleichstrom-Elektromotors vorgesehen sind.

16. Erfindung nach Anspruch 15, bei der die Mittel zur Umsteuerung eine Gegen-EMK-Steuerschaltung umfassen.

17. Erfindung nach Anspruch 3, bei der die Aktivierungsflüssigkeitspumpmittel eine Pumpe (320) und dafür einen Antriebsmotor (321) sowie ein Dreiwegeventil (350) umfassen, das mit dem Pumpenauslaß für einen abwechselnden Anschluß des Pumpenauslasses zu der einen oder der anderen der Aktivierungskammern (116, 216) verbunden ist.

18. Erfindung nach Anspruch 17, bei der die Pumpe (320) eine Hochgeschwindkeits-Mischstrom-Rotorpumpe ist.

19. Erfindung nach Anspruch 17, bei der der Antriebsmotor (321) ein bürstenloser Gleichstrom-Elektromotor ist.

20. Erfindung nach Anspruch 19, bei der außerdem Mittel zur Umsteuerung des bürstenlosen Gleichstrom-Elektromotors vorgesehen sind.

21. Erfindung nach Anspruch 20, bei der die Mittel zur Umsteuerung eine Gegen-EMK-Steuerschaltung umfassen.

22. Erfindung nach Anspruch 17, bei der das Dreiwegeventil (350) eien gesonderten Antriebsmotor (351) dafür aufweist.

23. Erfindung nach Anspruch 2 oder 3, bei der der Bluteinlaß (113, 213) und der Blutauslaß (115, 215) mit prothetischen Ventilen versehen sind, die so eingerichtet sind, daß sie den Blutstrom nur in einer Richtung gestatten.

24. Erfindung nach Anspruch 2 oder 3, bei der die Blutpumpenkammer (114, 214) eine Zone variablen Volumens umfaßt, die teilweise durch eine flexible Membrane (112, 212) aus einem biokompatiblen, elastomerischen Material und teilweise durch ein steifes Blutpumpenkammergehäuse (110, 210) festgelegt ist.

25. Erfindung nach Anspruch 1, 2 oder 3, bei der die Aktivierungskammer (116, 216) eine Zone variablen Volumens aufweist, die teilweise durch einen flexiblen Bereich (112, 212) der Blutpumpenkammer (114, 214) und teilweise durch ein steifes Aktivierungskammergehäuse (110, 210) festgelegt ist.

26. Erfindung nach Anspruch 1, 2 oder 3, bei der die Ventilmittel so gestaltet sind:

daß der Aktivierungsflüssigkeitsauslaß (132, 232) zu Beginn jedes Flüssigkeitsimpulses schließt, wenn ein genügender Strom von Aktivierungsflüssigkeit zu dem Aktivierungsflüssigkeitseinlaß (124, 224) besteht, um Kräfte oberhalb einer vorgegebenen Schwellgröße zu erzeugen;

daß der Aktivierungsflüssigkeitsauslaß (132, 232) während der Impulse geschlossen gehalten wird, während der Strom durch den Einlaß ausreichend ist, um die Kräfte oberhalb der vorgebenen Schwellengröße zu halten; und

daß der Aktivierungsflüssigkeitsauslaß (132, 232) am Ende der Impulse geöffnet wird, wenn der Strom durch den Einlaß (124, 224) auf einen Wert reduziert ist, bei dem die Kräfte unter die vorgegebene Schwellengröße fallen.

27. Erfindung nach Anspruch 26, bei der die Ventilmittel Federmittel (250) zur Ausübung einer permanenten Vorspannkraft umfassen, die die Tendenz hat, den Aktivierungsflüssigkeitsauslaß (132, 232) zu öffnen, wobei die Größe der Vorspannkraft gleich der vorgegebenen Schwelle ist.

28. Erfindung nach Anspruch 27, bei der die Ventilmittel so ausgebildet sind, daß sie in Abhängigkeit der Druckkräfte wirken, die durch den Strom der Aktivierungsflüssigkeit durch den Einlaß (124, 224, 242) bewirkt werden.

29. Erfindung nach Anspruch 28, bei der die Ventilmittel ein hohles Schließglied (330, 430) umfassen, dessen Inneres in Flüssigkeitsverbindung mit Aktivierungsflüssigkeit aus den Pumpmitteln steht und das zwischen einer geschlossenen Position, in der der Aktivierungsflüssigkeitsauslaß (132, 232) verschlossen ist, wenn dieser während der Flüssigkeitsimpulse mit Aktivierungsflüssigkeit gefüllt wird, und einer offenen Position im Abstand von dem Aktivierungsflüssigkeitsauslaß (132, 232) zwischen Flüssigkeitsimpulsen bewegbar ist.

30. Erfindung nach Anspruch 29, bei der das Schließglied (330, 430) eine expandierbare Hülle umfaßt.

31. Erfindung nach Anspruch 30, bei der die Hülle elastisches Material enthält.

32. Erfindung nach Anspruch 27, bei der die Ventilmittel ein Schließglied (430, 246) umfassen, das zwischen einer geschlossenen Position, in der der Flüssigkeitsauslaß (132, 232) während der Flüssigkeitsimpulse verschlossen ist, und einer offenen Position in einem Abstand von dem Aktivierungsflüssigkeitsauslaß (132, 232) zwischen den Flüssigkeitsimpulsen beweglich ist.

33. Erfindung nach Anspruch 32, bei der die Ventilmittel so beschaffen sind, daß sie in Abhängigkeit von Differenzdruckkräften wirken.

34. Erfindung nach Anspruch 33, bei der der Flüssigkeitseinlaß mindestens eine Mündung (432, 254) in dem Schließglied (430, 246) einschließt, durch die Aktivierungsflüssigkeit von den Pumpmitteln in die Aktivierungskammer (116, 216) strömt.

35. Erfindung nach Anspruch 34, bei der das Schließglied (430) hohl ist und dessen Inneres in Flüssigkeitsverbindung mit Aktivierungsflüssigkeit steht, die von den Pumpmitteln geliefert wird, wobei die Mündung (432) innerhalb der Wandung des hohlen Schließgliedes gebildet ist.

36. Erfindung nach Anspruch 35, bei der das Schließglied (430) eine expandierbare Hülle einschließt.

37. Erfindung nach Anspruch 36, bei der die Mündung innerhalb eines im wesentlichen steifen Wandabschnittes der Hülle gebildet ist.

38. Erfindung nach Anspruch 37, bei der die Hülle elastomerisches Material enthält.

39. Erfindung nach Anspruch 36, bei der die Hülle elastomerisches Material enthält.

40. Erfindung nach Anspruch 34, die außerdem Mittel (440, 256) zur Beibehaltung eines im wesentlichen konstanten Druckgefälles zu beiden Seiten des Schließgliedes (430, 246) während der Flüssigkeitsimpulse umfaßt trotz Änderung der Fließrate der Flüssigkeit in dem Flüssigkeitseinlaß.

41. Erfindung nach Anspruch 40, bei der die Mittel zur Beibehaltung des im wesentlichen konstanten Druckgefälles mindestens eine Öffnung (254) in dem Schließglied umfassen und außerde Schließmittel (440, 256), die vorgespannt sind, um die Öffnung im wesentlichen zu verschließen, jedoch bewegbar sind, um die Öffnung in einem Maße zu öffnen, das proportional der Fließrate der Flüssigkeit in dem Flüssigkeitseinlaß ist.

42. Erfindung nach Anspruch 41, bei der die Schließmittel einen Blattfederschließer (256), der über der Öffnung (254) liegt, umfassen.

43. Erfindung nach Anspruch 41, bei der das Schließglied (430) hohl ist und dessen Inneres in Flüssigkeitsverbindung mit Aktivierungsflüssigkeit steht, die von den Pumpmitteln geliefert wird, wobei die Öffnung (432) innerhalb der Wandung des hohlen Schließgliedes gebildet ist.

44. Erfindung nach Anspruch 43, bei der das Schließglied (430) eine expandierbare Hülle einschließt.

45. Erfindung nach Anspruch 44, bei der die Mündung (432) und die Öffnung innerhalb eines im wesentlichen steifen Wandabschnittes (434) der Hülle gebildet sind.

46. Erfindung nach Anspruch 45, bei der die Hülle elastomerisches Material enthält.

47. Erfindung nach Anspruch 44, bei der die Hülle elastomerisches Material enthält.

48. Erfindung nach Anspruch 2 oder 3, die außerdem Mittel (C₁, C₂) zur Aufbringung einer elektrischen Leistung auf die Aktivierungsflüssigkeitspumpmittel und die damit verbundene Steuerelektronik (C) innerhalb eines Patienten umfaßt, der eine eingepflanzte Herzprothese trägt.

49. Erfindung nach Anspruch 48, bei der die Mittel zur Einspeisung elektrischer Leistung eine externe Primärspule ($C_1$) umfassen, die induktiv mit einer unter der Haut befindliche Sekundärspule ($C_2$) zur Übertragung elektrischer Energie durch die unversehrte Haut des Patienten verbunden ist.

50. Erfindung nach Anspruch 33, bei der die Aktivierungskammer (216) einen Verteiler (240) und ein Fließglied (244) einschließt, das das Innere des Verteilers (240) und das Innere der Aktivierungskammer (216) miteinander verbindet und durch das Aktivierungsflüssigkeit hindurchfließt, wenn sich die Aktivierungskammer (216) füllt, wobei der Aktivierungsflüssigkeitseinlaß und -flüssigkeitsauslaß in der Wand des Verteilers gebildet sind und das Schließglied (246) sich innerhalb des Fließgliedes (244) befindet und dieses teilweise verschließt sowie innerhalb des Fließgliedes in die geschlossene Position zu Beginn jedes Flüssigkeitsimpulses beweglich ist, wenn die Differenzdruckkraft zu beiden Seiten des Schließgliedes die Kraftgröße der Federmittel (250) übersteigt.

51. Erfindung nach Anspruch 50, bei der das Schließglied (246) scharnierartig aufgehängt ist und zwischen der geschlossenen und offenen Lage eine Schwenkbewegung vollführt.

52. Erfindung nach Anspruch 51, bei der die Federmittel eine Blattfeder (250) umfassen, die an dem einen Ende an der Wand des Verteilers (240) befestigt ist und nach Art eines Scharniers das Schließglied (246) an dem anderen Ende hält.

53. Erfindung nach Anspruch 51, bei der der Aktivierungsflüssigkeitsauslaß (232) an der einen Seite und gegenüberliegend von dem Fließglied (244) angeordnet ist, das Schließglied einen Schließer (246) aufweist, das sich über den Aktivierungsflüssigkeitsauslaß (232) bewegen kann und einen Schwingarm (250) enthält, der an dem einen Ende an dem Schließer (246) befestigt ist, sich über das Fließglied erstreckt und an seinem gegenüberliegenden Ende an der Wand des Verteilers (240) auf der gegenüberliegenden Seite des Fließgliedes (244) befestigt ist.

54. Erfindung nach Anspruch 53, bei der der Schwingarm (250) das Federmittel ist und eine Blattfeder umfaßt, die den Schließer (246) nach Art eines Scharnieres trägt.

55. Erfindung nach Anspruch 54, bei der das Schließglied (246) mit Mitteln zur Beibehaltung eines im wesentlichen konstanten Druckabfalles über das Schließmittel (246) trotz Änderungen in der Fließrate der Flüssigkeit in den Verteiler (240) hinein versehen ist.

56. Erfindung nach Anspruch 55, bei der die Mittel zur Beibehaltung eines relativ konstanten Druckabfalls aus einem Blattfederschließer (256) bestehen, der vorgespannt ist, um im wesentlichen die Mündung (254) zu schließen, jedoch geöffnet werden kann, um die Mündung in einem Maße zu öffnen, das proportional der Fließrate der Flüssigkeit in den Verteiler (240) hinein ist.

## Revendications

1. Un système d'actionnement hydraulique implantable pour l'alimentation en force motrice d'une chambre de pompage de sang (114, 214) ayant une portion flexible (112, 212) comprenant: un réservoir de fluide d'actionnement (12); des moyens de pompage de fluide d'actionnement (120, 220, 320) en communication fluide avec ledit réservoir pour délivrer des pulsations intermittentes de fluide d'actionnement; et une chambre d'actionnement (116, 216) ayant une voie d'entrée de fluide d'actionnement (124, 224) en communication fluide avec les organes de pompage cités, ladite chambre d'actionnement étant adaptée pour provoquer le déplacement de la portion flexible de ladite chambre de pompage du sang en réaction aux variations de volume du fluide d'actionnement dans ladite chambre d'actionnement, caractérisé par une sortie (132, 232) de la chambre d'actionnement (116, 216) pour relier cette chambre au réservoir de fluide (12) pendant la période de décharge et par des organes du type valve (130, 230, 330, 430, 446) pour fermer la sortie pendant la période d'actionnement, la force de fermeture des organes du type valve étant générée par le débit de fluide à travers ladite voie d'entrée de fluide d'actionnement.

2. Un système de pompage de sang actionné hydrauliquement implantable comprenant une chambre de pompage de sang (114, 214) ayant une entrée de sang (113, 213), une sortie de sang (115, 215) et une portion flexible (112, 212); un réservoir de fluide d'actionnement (12); des moyens de pompage de fluide d'actionnement (120, 220, 320) en communication fluide avec ledit réservoir pour délivrer des pulsations intermittentes de fluide d'actionnement; et une chambre d'actionnement (116, 216) ayant une voie d'entrée de fluide d'actionnement (124, 224) en communication fluide avec les organes de pompage cités, le fonctionnement de ladite chambre d'actionnement étant associé à celui de ladite chambre de pompage du sang pour provoquer le déplacement de ladite portion flexible de ladite chambre de pompage du

3. Une prothèse cardiaque totale actionnée hydrauliquement implantable comprenant une paire de chambres de pompage de sang (114, 214), chacune ayant une entrée de sang (113, 213), une sortie de sang (115, 215) et une portion flexible (112, 212); une paire de chambres d'actionnement (116, 216), chacune d'elles ayant une voie d'entrée de fluide d'actionnement (124, 224), le fonctionnement de l'une des chambres d'actionnement citées étant associé à celui de l'une des chambres de pompage de sang citées et le fonctionnement de l'autre chambre d'actionnement citée étant associé à celui de l'autre chambre de pompage de sang citée pour provoquer le déplacement de la portion flexible des chambres de pompage de sang citées en réaction aux variations de volume du fluide d'actionnement dans leurs chambres d'actionnement associées;

un réservoir de fluide d'actionnement (12) et des organes de pompage de fluide d'actionnement (120, 220) en communication fluide avec ledit réservoir (12) et les voies d'entrée de fluide d'actionnement citées (124, 224) pour délivrer des pulsations intermittentes de fluide d'actionnement à partir dudit réservoir aux chambres d'actionnement citées, caractérisée par une sortie (132, 232) de la chambre d'actionnement (116, 216) pour relier cette chambre au réservoir de fluide (12) pendant la période de décharge et par des organes du type valve (130, 230, 330, 430, 446) pour fermer la sortie pendant la période d'actionnement, la force de fermeture des organes du type valve étant générée par le débit de fluide à travers ladite voie d'entrée de fluide d'actionnement, ladite chambre d'actionnement se remplissant de fluide d'actionnement et le sang étant refoulé de ladite chambre de pompage de sang associée (114, 214), tandis que ladite valve est fermée, et le fluide d'actionnement s'écoulant de ladite chambre d'actionnement permettant à ladite chambre de pompage de sang associée de se remplir de sang, tandis que ladite valve est ouverte.

4. L'invention de la revendication 1, 2 ou 3 dans laquelle ledit réservoir de fluide d'actionnement (12) comprend une zone souple délimitée au moins partiellement par une portion de paroi flexible (112, 212) en élastomère bio-compatible.

5. L'invention de la revendication 4 dans laquelle ledit réservoir de fluide d'actionnement (12) comprend également des tuyaux flexibles (122, 222) menant vers et partant des organes de pompage de fluide d'actionnement cités (120, 220, 320) et menant vers et partant de ladite chambre d'actionnement (116, 216).

6. L'invention de la revendication 3 dans laquelle ledit réservoir de fluide d'actionnement (12) comprend une zone souple séparée associée à chacune des chambres d'actionnement citées (116, 216) et délimitée au moins partiellement par une portion de paroi flexible (10) en élastomère biocompatible, les zones souples citées communicant les unes avec les autres, avec les organes de pompage cités (120, 220, 320) et les chambres d'actionnement citées (116, 216) par des tuyaux flexibles (124, 224, 122, 222).

7. Les inventions de la revendication 1 ou 2 dans lesquelles les organes de pompage du fluide d'actionnement cités comprennent une pompe (120, 220, 320) et un moteur de commande (121, 221, 321) pour celle-ci.

8. L'invention de la revendication 7 dans laquelle la pompe (121, 221, 321) est une pompe rotative à pignons à grande vitesse.

9. L'invention de la revendication 7 dans laquelle ledit moteur de commande (121, 221, 321) est un moteur électrique à courant continu sans balais.

10. L'invention de la revendication 9 qui comprend en plus des moyens de commutation du moteur électrique à courant continu sans balais cité.

11. L'invention de la revendication 10 dans laquelle les moyens de commutation cités comprennent des lignes de commande d'énergie électromotrice de retour.

12. L'invention de la revendication 3 dans laquelle les moyens de pompage de fluide d'actionnement cités comprennent une pompe séparée (120, 220) et un moteur de commande (121, 221) pour celle-ci associés à chacune des chambres d'actionnement citées (116, 216).

13. L'invention de la revendication 12 dans laquelle chacune des pompes citées (120, 220) est une pompe rotative à pignons à grande vitesse.

14. L'invention de la revendication 12 dans laquelle chacun des moteurs de commande cités (121, 221) est un moteur électrique à courant continu sans balais.

15. L'invention de la revendication 14 qui comprend en plus des moyens de commutation du moteur électrique à courant continu sans balais sus-cité.

16. L'invention de la revendication 15 dans laquelle les moyens de commutation cités comprennent des lignes de commande d'énergie électromotrice de retour.

17. L'invention de la revendication 3 dans laquelle les organes de pompage de fluide d'actionnement cités comprennent une pompe (320), un moteur de commande (321) pour celle-ci et une vanne d'aiguillage à trois voies (350) associée à la sortie de la pompe pour diriger le débit de la pompe en alternance vers l'une ou l'autre chambre d'actionnement (116, 216).

18. L'invention de la revendication 17 dans laquelle ladite pompe (320) est une pompe rotative à pignons à grande vitesse.

19. L'invention de la revendication 17 dans laquelle ledit moteur de commande (321) est un moteur électrique à courant continu sans balais.

20. L'invention de la revendication 19 qui comprend en plus des moyens de commutation du moteur électrique à courant continu sans balais cité.

21. L'invention de la revendication 20 dans laquelle les moyens de commutation cités comprennent des lignes de commande d'énergie électromotrice de retour.

22. L'invention de la revendication 17 dans laquelle ladite vanne d'aiguillage à trois voies (350) a un moteur de commande (351) séparé qui lui est associé.

23. L'invention de la revendication 2 ou 3 dans laquelle ladite entrée du sang (113, 213) et ladite sortie du sang (115, 215) sont équipées de valves prothétiques adaptées pour permettre le flux de sang dans une direction seulement.

24. L'invention de la revendication 2 ou 3 dans laquelle ladite chambre de pompage du sang (114, 214) comprend une zone à volume variable délimitée en partie par une membrane flexible (112, 212) en élastomère biocompatible et en partie par un bâti rigide de chambre de pompage de sang (110, 210).

25. L'invention de la revendication 1, 2 ou 3 dans laquelle ladite chambre d'actionnement (116, 216) comprend une zone à volume variable délimitée en partie par ladite portion flexible (112, 212) de ladite chambre de pompage de sang (114, 214) et en partie par un bâti rigide de chambre d'actionnement (110, 210).

26. L'invention de la revendication 1, 2 ou 3 dans laquelle la valve est adaptée:

— pour fermer ladite voie de sortie (132, 232) du fluide d'actionnement au début de chacune des pulsations de fluide citées, si un débit suffisant de fluide d'actionnement est délivré à ladite voie d'entrée (124, 224) du fluide d'actionnement pour créer des forces supérieures à un seuil d'une valeur prédéfinie;

— pour maintenir ladite voie de sortie (132, 232) du fluide d'actionnement fermée pendant les pulsations citées, tandis que ledit débit à travers ladite voie d'entrée (124, 224) est suffisant pour maintenir les forces citées au-dessus dudit seuil d'une valeur prédéfinie et

— pour ouvrir ladite voie de sortie (132, 232) du fluide d'actionnement à la fin des pulsations citées, lorsque ledit débit à travers la voie d'entrée (124, 224) est réduit à une valeur à laquelle les forces citées tombent au-dessous du seuil d'une valeur prédéfinie.

27. L'invention de la revendication 26 dans laquelle la valve comprend un ressort (250) qui exerce une force permanente de précontrainte qui tend à ouvrir ladite voie de sortie (132, 232) de fluide d'actionnement, l'ordre de grandeur de ladite force de précontrainte étant égal au seuil prédéfini cité.

28. L'invention de la revendication 27 dans laquelle ladite valve est adaptée pour agir en réponse aux forces de pression créées par le débit du fluide d'actionnement à travers ladite voie d'entrée (124, 224, 242).

29. L'invention de la revendication 28 dans laquelle ladite valve comprend un organe de fermeture creux (330, 430) ayant l'intérieur en communication fluide avec le fluide d'actionnement délivré par les organes de pompage cités et mobile entre une position fermée qui bloque ladite voie de sortie (132, 232) de fluide d'actionnement lorsqu'elle est pleine de fluide d'actionnement pendant les pulsations de fluide et une position ouverte libre de ladite voie de sortie (132, 232) du fluide d'actionnement entre les pulsations.

30. L'invention de la revendication 29 dans laquelle ledit organe de fermeture (330, 430) contient une enveloppe extensible.

31. L'invention de la revendication 30 dans laquelle ladite enveloppe contient de l'élastomère.

32. L'invention de la revendication 27 dans laquelle ladite valve comprend un organe de fermeture (430, 246) mobile entre une position fermée qui bloque ladite voie de sortie (132, 232) pendant les pulsations de fluide et une position ouverte libre de ladite voie de sortie (132, 232) entre les pulsations de fluide.

33. L'invention de la revendication 32 dans laquelle la valve est adaptée pour agir en réaction aux forces de pression différentielle.

34. L'invention de la revendication 33 dans laquelle ladite voie d'entrée de fluide comprend au moins un orifice (432, 254) à travers l'organe de fermeture (430, 246) par lequel le fluide d'actionnement s'écoule des organes de pompage cités dans ladite chambre d'actionnement (116, 216).

35. L'invention de la revendication 34 dans laquelle l'organe de fermeture (430) est creux et a l'intérieur en communication fluide avec le fluide d'actionnement délivré par les organes de pompage cités et ledit orifice (432) est formé à travers la paroi de l'organe creux de fermeture cité.

36. L'invention de la revendication 35 dans laquelle l'organe de fermeture (430) comprend une enveloppe extensible.

37. L'invention de la revendication 36 dans laquelle ledit orifice est formé à travers une portion de paroi rigide en substance de ladite enveloppe.

38. L'invention de la revendication 37 dans laquelle ladite enveloppe contient de l'élastomère.

39. L'invention de la revendication 36 dans laquelle ladite enveloppe contient l'élastomère.

40. L'invention de la revendication 34 qui comprend en plus des organes (440, 256) pour maintenir une pression différentielle constante en substance à travers ledit organe de fermeture (430, 246) pendant les pulsations de fluide en dépit des variations de débit de fluide le long de ladite voie d'entrée du fluide.

41. L'invention de la revendication 40 dans laquelle les organes cités pour maintenir une pression différentielle constante en substance contiennent au moins une ouverture (254) à travers ledit organe de fermeture et des organes de fermeture (440, 256) précontraints pour fermer en substance ladite ouverture mais mobiles pour ouvrir ladite ouverture proportionnellement au débit de fluide le long de ladite voie d'entrée de fluide.

42. L'invention de la revendication 41 dans laquelle les organes de fermeture cités contiennent une fermeture à ressort à lame (256) qui recouvre ladite ouverture (254).

43. L'invention de la revendication 41 dans laquelle l'organe de fermeture (430) est creux et a l'intérieur en communication fluide avec le fluide d'actionnement délivré par les organes de pompage cités, ledit orifice (432) et ladite ouverture étant formés à travers la paroi de l'organe creux de fermeture cité (430).

44. L'invention de la revendication 43 dans laquelle ledit organe de fermeture (430) contient une enveloppe extensible.

45. L'invention de la revendication 44 dans laquelle ledit orifice (432) et ladite ouverture sont formés à travers une portion de paroi (434) rigide en substance de ladite enveloppe.

46. L'invention de la revendication 45 dans laquelle l'enveloppe contient de l'élastomère.

47. L'invention de la revendication 44 dans laquelle l'enveloppe contient de l'élastomère.

48. L'invention de la revendication 2 ou 3 qui comprend en plus des organes ($C_1$, $C_2$) pour appliquer l'énergie électrique aux organes de pompage de fluide d'actionnement cités aux organes de pompage de fluide d'actionnement cités et une électronique de contrôle associée (C) chez un patient ayant une prothèse cardiaque implantée.

49. L'invention de la revendication 48 dans laquelle les organes cités pour délivrer l'énergie électrique contiennent une bobine primaire externe ($C_1$) accouplée inductivement à une bobine secondaire sous-cutanée ($C_2$) pour la transmission de l'énergie électrique à travers la peau intacte du patient cité.

50. L'invention de la revendication 33 dans laquelle ladite chambre d'actionnement (216) comprend un répartiteur (240) et un canal d'entrée du débit (244) traversant l'intérieur dudit répartiteur (240) et l'intérieur de ladite chambre d'actionnement (216) par lequel passe le fluide d'actionnement lorsqu'il remplit ladite chambre d'actionnement (216), les voies d'entrée et de sortie du fluide d'actionnement citées étant formées dans la paroi dudit répartiteur, ledit organe de fermeture (246) étant disposé à l'intérieur et bloquant partiellement le canal d'entrée de débit (244) et étant mobile à l'intérieur dudit canal de débit vers sa position fermée au début de chaque pulsation de fluide, lorsque la force de pression différentielle à travers ledit organe de fermeture dépasse l'ordre de grandeur de la force du ressort cité (250).

51. L'invention de la revendication 50 dans laquelle ledit organe de fermeture (246) est pivotable et est soumis à un pivotement entre les positions fermée et ouverte citées.

52. L'invention de la revendication 51 dans laquelle ledit ressort contient un ressort à lame (250) fixé à une extrémité à la paroi du répartiteur cité (240) et portant de manière pivotante ledit organe de fermeture (246) à l'autre extrémité.

53. L'invention de la revendication 51 dans laquelle ladite voie de sortie (232) de fluide d'actionnement est disposée sur l'un des côtés du canal de débit cité (244) de manière transversale par rapport à celui-ci, ledit organe de fermeture contient une fermeture (246) mobile à travers ladite sortie (232) de fluide d'actionnement et une petite bascule (250) reliée à une extrémité à ladite fermeture (246) s'étendant à travers ledit canal de débit et fixée à son extrémité opposée à la paroi du répartiteur (240) cité sur le côté opposé du canal de débit cité (244).

54. L'invention de la revendication 53 dans laquelle la petite bascule (250) est appelée ressort et contient un ressort à lame qui supporte ladite fermeture (246) de manière pivotante.

55. L'invention de la revendication 54 dans laquelle l'organe de fermeture (246) est équipé d'organes pour maintenir une chute de pression constante en substance à travers ledit organe de fermeture (246) en dépit des variations de débit de fluide dans ledit répartiteur (240).

56. L'invention de la revendication 55 dans laquelle l'organe pour maintenir une chute de pression relativement constante comprend une fermeture à ressort à lame (256) précontrainte pour fermer en substance ledit orifice (254) qui est mobile pour ouvrir ledit orifice proportionnellement par rapport au débit de fluide dans le répartiteur (240).

FIG.1

IMPLANTED COMPONENTS

INTERNAL BATTERIES — IB

SKIN

$C_2$    $C_1$

P — PULMONARY CIRCULATION

RV — RIGHT VENTRICLE (BLOOD PUMP)

RA — HYDRAULIC ACTUATOR

C — INTERNAL ELECTRONIC CONTROLS, POWER, & MONITORING

S — SYSTEMIC CIRCULATION

LV — LEFT VENTRICLE (BLOOD PUMP)

LA — HYDRAULIC ACTUATOR

EXTERNAL WEARABLE COMPONENTS

EB — EXTERNAL BATTERY

PC — EXTERNAL ELECTRONIC CONTROLS, POWER, & MONITORING

AC OR DC POWER

0 079 373

FIG. 2

FIG.12

FIG.13

FIG. 3

FIG. 4

FIG. 5

FIG. 6

FIG. 7

FIG. 8

FIG.9

FIG.10

FIG.11